# EUROPEAN PATENT SPECIFICATION

(11) **EP 1 765 152 B1**
(45) Date of publication and mention of the grant of the patent: **04.01.2017**
(21) Application number: 05758748.7
(22) Date of filing: 03.06.2005
(51) Int. Cl.: A61B 5/00, A61B 17/14, A61B 17/32, B65D 81/00

(54) **APPARATUS FOR AN INTEGRATED SAMPLE CAPTURE AND ANALYSIS DISPOSABLE**
GERÄT FÜR EINEN INTEGRIERTEN EINWEGARTIKEL ZUR PROBENERFASSUNG UND AUSWERTUNG
APPAREIL JETABLE POUR ANALYSE ET CAPTURE D'ECHANTILLON INTEGRE

(30) Priority: 03.06.2004 US 576998 P
(43) Date of publication of application: 28.03.2007
(73) Proprietor: Sanofi-Aventis Deutschland GmbH, 65929 Frankfurt am Main (DE)
(72) Inventor: MAUZE, Ganapati, Sunnyvale, CA 94087 (US); LUM, Paul, Los Altos, CA 94022 (US); DESHMUKH, Ajay, Mountain View, CA 94041 (US); MARSOT, Travis, Palo Alto, CA 94303 (US); BOECKER, Dirk, Palo Alto, CA 94306 (US); FREEMAN, Dominique, M., La Honda, CA 94020 (US); ALDEN, Don, Sunnyvale, CA 94087 (US); CANE, Michael, Church Road Toft Cambridge CB3 7RF (GB); SCHUMANN, Matt, Cambridge CB3 7RY (GB); ROSS, James, Livermore, CA 94551 (US)
(74) Representative: McDougall, James
(86) International application number: PCT/US2005/019590
(87) International publication number: WO 2005/120199

(56) References cited:
- WO-A-01/95806
- US-A1- 2003 199 789
- US-A1- 2003 199 893
- US-A1- 2005 021 066
- US-B1- 6 251 083
- US-B2- 6 706 159
- US-B2- 6 706 159
- US-B2- 6 783 502

## Description

### BACKGROUND OF THE INVENTION

### Technical Field:

The technical field relates to analyte detecting devices, and more specifically, coatings for improving glucose measurement.

### Background Art:

Lancing devices are known in the medical health-care products industry for piercing the skin to produce blood for analysis. Typically, a drop of blood for this type of analysis is obtained by making a small incision in the fingertip, creating a small wound, which generates a small blood droplet on the surface of the skin.

Early methods of lancing included piercing or slicing the skin with a needle or razor. Current methods utilize lancing devices that contain a multitude of spring, cam and mass actuators to drive the lancet. These include cantilever springs, diaphragms, coil springs, as well as gravity plumbs used to drive the lancet. The device may be held against the skin and mechanically triggered to ballistically launch the lancet. Unfortunately, the pain associated with each lancing event using known technology discourages patients from testing. In addition to vibratory stimulation of the skin as the driver impacts the end of a launcher stop, known spring based devices have the possibility of firing lancets that harmonically oscillate against the patient tissue, causing multiple strikes due to recoil. This recoil and multiple strikes of the lancet is one major impediment to patient compliance with a structured glucose monitoring regime.

Success rate generally encompasses the probability of producing a blood sample with one lancing action, which is sufficient in volume to perform the desired analytical test. The blood may appear spontaneously at the surface of the skin, or may be "milked" from the wound. Milking generally involves pressing the side of the digit, or in proximity of the wound to express the blood to the surface. In traditional methods, the blood droplet produced by the lancing action must reach the surface of the skin to be viable for testing.

When using existing methods, blood often flows from the cut blood vessels but is then trapped below the surface of the skin, forming a hematoma. In other instances, a wound is created, but no blood flows from the wound. In either case, the lancing process cannot be combined with the sample acquisition and testing step. Spontaneous blood droplet generation with current mechanical launching system varies between launcher types but on average it is about 50% of lancet strikes, which would be spontaneous. Otherwise milking is required to yield blood. Mechanical launchers are unlikely to provide the means for integrated sample acquisition and testing if one out of every two strikes does not yield a spontaneous blood sample.

Many diabetic patients (insulin dependent) are required to self-test for blood glucose levels five to six times daily. The large number of steps required in traditional methods of glucose testing ranging from lancing, to milking of blood, applying blood to the test strip, and getting the measurements from the test strip discourages many diabetic patients from testing their blood glucose levels as often as recommended. Tight control of plasma glucose through frequent testing is therefore mandatory for disease management. The pain associated with each lancing event further discourages patients from testing. Additionally, the wound channel left on the patient by known systems may also be of a size that discourages those who are active with their hands or who are worried about healing of those wound channels from testing their glucose levels.

Another problem frequently encountered by patients who must use lancing equipment to obtain and analyze blood samples is the amount of manual dexterity and hand-eye coordination required to properly operate the lancing and sample testing equipment due to retinopathies and neuropathies particularly, severe in elderly diabetic patients. For those patients, operating existing lancet and sample testing equipment can be a challenge. Once a blood droplet is created, that droplet must then be guided into a receiving channel of a small test strip or the like. If the sample placement on the strip is unsuccessful, repetition of the entire procedure including re-lancing the skin to obtain a new blood droplet is desired.

Early methods of using test strips required a relatively substantial volume of blood to obtain an accurate glucose measurement. This large blood requirement made the monitoring experience a painful one for the user since the user may need to lance deeper than comfortable to obtain sufficient blood generation. Alternatively, if insufficient blood is spontaneously generated, the user may need to "milk" the wound to squeeze enough blood to the skin surface. Neither method is desirable as they take additional user effort and may be painful. The discomfort and inconvenience associated with such lancing events may deter a user from testing their blood glucose levels in a rigorous manner sufficient to control their diabetes.

A further impediment to patient compliance is the amount of time that at lower volumes, it becomes even more important that blood or other fluid sample be directed to a measurement device without being wasted or spilled along the way. Known devices do not effectively handle the low sample volumes in an efficient manner. Accordingly, improved sensing devices are desired to increase user compliance and reduce the hurdles associated with analyte measurement.

US 2003/199893 A1 discloses a device for use with a penetrating member driver to penetrate tissue. A plurality of penetrating members are coupled to a single cartridge and are operatively couplable to the penetrating member driver. The penetrating members are movable to extend radially outward from the cartridge to penetrate tissue. A plurality of analyte sensors are coupled to the single cartridge and are positioned on the cartridge to receive body fluid from a wound in the tissue created by the penetrating member.

### SUMMARY OF THE INVENTION

Aspects of the invention are set out in the appended claims.

The present invention provides solutions for at least some of the drawbacks discussed above. Specifically, some embodiments of the present invention provide an improved apparatus for measuring analyte levels in a body fluid. At least some of these and other objectives described herein will be met by embodiments of the present invention.

In one embodiment a device is provided comprising a cartridge having a plurality of cavities; a plurality of analyte detecting members coupled to the cartridge; and a plurality of sample capture devices.

In another embodiment a device is provided comprising a cartridge having a plurality of cavities; a plurality of penetrating members at least partially contained in the cavities of the single cartridge wherein the penetrating members are slidably movable to extend outward from lateral openings on the cartridge to penetrate tissue; a sterility barrier coupled to the cartridge, the sterility barrier covering a plurality of the lateral openings, wherein the sterility barrier covering the lateral openings is configured to be moved so that a penetrating member exits the lateral opening without contacting the barrier; a plurality of analyte detecting members coupled to an underside of the cartridge; and a plurality of sample capture devices, the sample capture devices each having a vertical portion and a horizontal portion, the vertical portion having a opening there through to allow a penetrating member to pass through.

The sample capture devices may be L shaped. The fluid flow path from the finger contact area to the analyte measurment members may be L-shaped. The sample capture devices may be formed on circular disc and coupled to the cartridge. The sample capture devices may each include a wicking member. The sample capture devices may each include a hydrophilic membrane. The sample capture devices may each include a wicking member and a capillary member. The sample capture devices may each have a lollipop shaped opening on the vertical portion. The sample capture devices may be individually movable to be in a valve open or a valve closed position. The sample capture devices may be hinged to a disc.

In another embodiment a method of manufacturing an analyte detecting device is provided. The method comprises providing a housing; providing a cartridge that is sized to fit within the housing; forming an opening on the housing; applying at least one layer of viscoelastic material on the housing around the opening, the material applying an compression force to a target tissue when the target tissue engages the material; providing a plurality of penetrating members in the cartridge; and providing a plurality of analyte detection devices in the cartridge.

The analyte detection devices may measure glucose levels in a sample fluid. The penetrating members may be lancets. The method may include placing a solenoid penetrating member driver in the housing. The viscoelastic material may be formed in the shape of an O-ring. The viscoelastic material may have a primary portion and a secondary portion, wherein the secondary portion engages a portion of the tissue away from a lancing site and compressing the tissue to drive blood towards the lancing site. The method may include providing a plurality of seals on the cartridge to maintain the penetrating members in a sterile condition prior to use. The method may include providing an LCD screen on the housing to provide information to the user during use. The method may include providing an LCD screen on the housing to provide information to the user during use.

A further understanding of the nature and advantages of the invention will become apparent by reference to the remaining portions of the specification and drawings.

### BRIEF DESCRIPTION OF THE DRAWINGS

Figure 1 illustrates an embodiment of a controllable force driver in the form of a cylindrical electric penetrating member driver using a coiled solenoid -type configuration.
Figure 2A illustrates a displacement over time profile of a penetrating member driven by a harmonic spring/mass system.
Figure 2B illustrates the velocity over time profile of a penetrating member driver by a harmonic spring/mass system.
Figure 2C illustrates a displacement over time profile of an embodiment of a controllable force driver.
Figure 2D illustrates a velocity over time profile of an embodiment of a controllable force driver.
Figure 3 is a diagrammatic view illustrating a controlled feed-back loop.
Figure 4 is a perspective view of a tissue penetration device having features of the invention.
Figure 5 is an elevation view in partial longitudinal section of the tissue penetration device of Figure 4.
Figure 6 shows a schematic of a penetrating member piercing skin.
Figure 7 shows an exploded perspective view of one embodiment of a device
Figures 8 and 9 show a side and top views of a device for sample capture
Figures 10 through 18 show embodiments relating to the measurement of finger forces placed against an aperture annulus.
Figure 19 and 20 show embodiments for sample capture.
Figure 21 and 22 show embodiments for sample capture.
Figures 23 and 24 show embodiments for foil punch devices.
Figure 25 shows a foil peeling device.
Figures 26 and 27 show elements to facilitate manufacture.

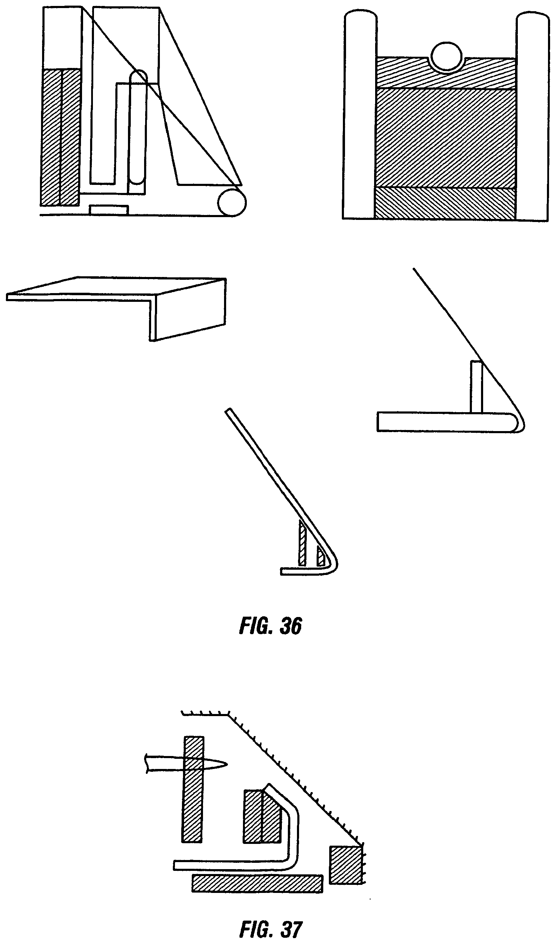

Figures 28 through 43 show various embodiments of punches.
Figures 44 through 51 relate to a sample capture structures
Figure 52 through 53 relate to controlling fluid access.
Figures 54 through 59 relate to sealing
Figures 60 through 61 relate to sealing
Figures 62 through 69 relate to punches and foils
Figure 70 is a schematic showing one embodiment of a manufacturing method
Figures 71 through 75 relate to manufacturing methods
Figures 76 through 80 relate to embodiments
Figures 81 through 88 relate to embodiments
Figure 89 through 107 relate to embodiment
Figures 108 to 134 relate to still further embodiments

### DESCRIPTION OF THE SPECIFIC EMBODIMENTS

The present invention provides a solution for body fluid sampling. The device may use a high density penetrating member design. It may use
penetrating members of smaller size, such as but not limited to diameter or length, than those of conventional penetrating members known in the art. The device may be used for multiple lancing events without having to remove a disposable from the device. The invention may provide improved sensing capabilities. At least some of these and other objectives described herein will be met by embodiments of the present invention.

It is to be understood that both the foregoing general description and the following detailed description are exemplary and explanatory only and are not restrictive of the invention, as claimed. It may be noted that, as used in the specification and the appended claims, the singular forms "a", "an" and "the" include plural referents unless the context clearly dictates otherwise. Thus, for example, reference to "a material" may include mixtures of materials, reference to "a chamber" may include multiple chambers, and the like.

In this specification and in the claims which follow, reference will be made to a number of terms which shall be defined to have the following meanings:
"Optional" or "optionally" means that the subsequently described circumstance may or may not occur, so that the description includes instances where the circumstance occurs and instances where it does not. For example, if a device optionally contains a feature for analyzing a blood sample, this means that the analysis feature may or may not be present, and, thus, the description includes structures wherein a device possesses the analysis feature and structures wherein the analysis feature is not present.

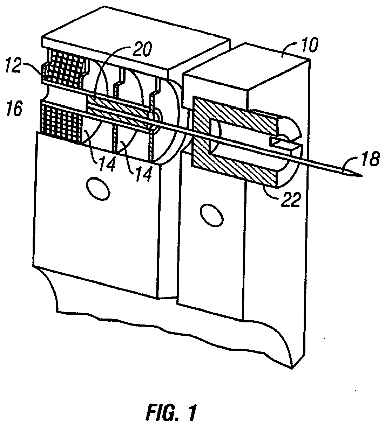

The device may be used with a variety of different penetrating member drivers. It is contemplated that these penetrating member drivers may be spring based, solenoid based, magnetic driver based, nanomuscle based, or based on any other mechanism useful in moving a penetrating member along a path into tissue. It should be noted that the disclosure is not limited by the type of driver used with the penetrating member feed mechanism. One suitable penetrating member driver for use with the present invention is shown in Figure 1. This is an embodiment of a solenoid type electromagnetic driver that is capable of driving an iron core or slug mounted to the penetrating member assembly using a direct current (DC) power supply. The electromagnetic driver includes a driver coil pack that is divided into three separate coils along the path of the penetrating member, two end coils and a middle coil. Direct current is alternated to the coils to advance and retract the penetrating member. Although the driver coil pack is shown with three coils, any suitable number of coils may be used, for example, 4, 5, 6, 7 or more coils may be used.

Referring to the embodiment of Figure 1, the stationary iron housing 10 may contain the driver coil pack with a first coil 12 flanked by iron spacers 14 which concentrate the magnetic flux at the inner diameter creating magnetic poles. The inner insulating housing 16 isolates the penetrating member 18 and iron core 20 from the coils and provides a smooth, low friction guide surface. The penetrating member guide 22 further centers the penetrating member 18 and iron core 20. The penetrating member 18 is protracted and retracted by alternating the current between the first coil 12, the middle coil, and the third coil to attract the iron core 20. Reversing the coil sequence and attracting the core and penetrating member back into the housing retracts the penetrating member. The penetrating member guide 22 also serves as a stop for the iron core 20 mounted to the penetrating member 18.

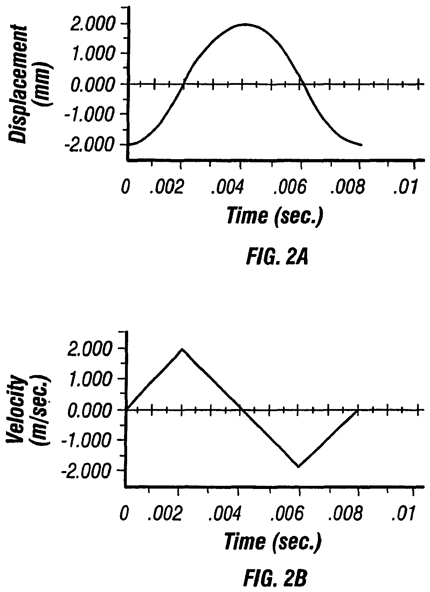

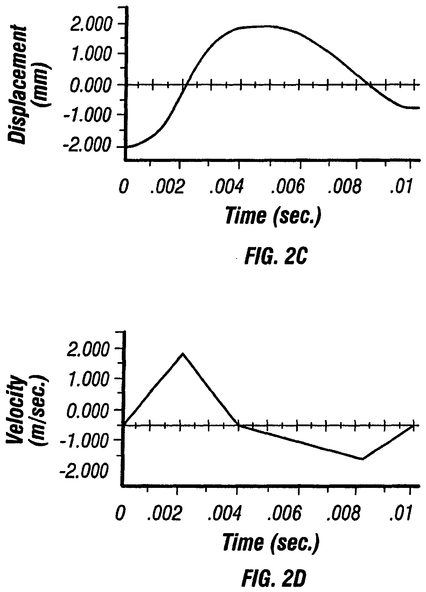

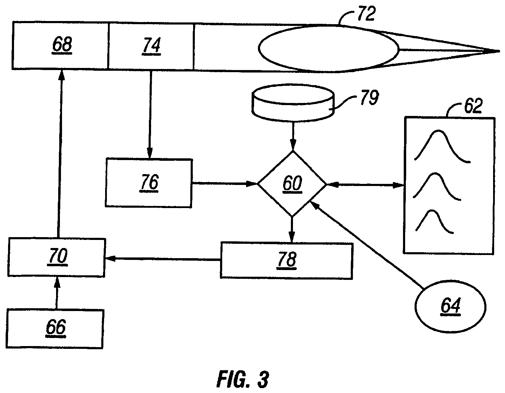

As discussed above, tissue penetration devices which employ spring or cam driving methods have a symmetrical or nearly symmetrical actuation displacement and velocity profiles on the advancement and retraction of the penetrating member as shown in Figures 2 and 3. In most of the available lancet devices, once the launch is initiated, the stored energy determines the velocity profile until the energy is dissipated. Controlling impact, retraction velocity, and dwell time of the penetrating member within the tissue can be useful in order to achieve a high success rate while accommodating variations in skin properties and minimize pain. Advantages can be achieved by taking into account of the fact that tissue dwell time is related to the amount of skin deformation as the penetrating member tries to puncture the surface of the skin and variance in skin deformation from patient to patient based on skin hydration.

In this embodiment, the ability to control velocity and depth of penetration may be achieved by use of a controllable force driver where feedback is an integral part of driver control. Such drivers can control either metal or polymeric penetrating members or any other type of tissue penetration element. The dynamic control of such a driver is illustrated in Figure. 2C which illustrates an embodiment of a controlled displacement profile and Figure 2D which illustrates an embodiment of a the controlled velocity profile. These are compared to Figures 2A and 2B, which illustrate embodiments of displacement and velocity profiles, respectively, of a harmonic spring/mass powered driver. Reduced pain can be achieved by using impact velocities of greater than about 2 m/s entry of a tissue penetrating element, such as a lancet, into tissue. Other suitable embodiments of the penetrating member driver are described in commonly assigned, copending U.S. Patent Application Ser. No. 10/127,395, (Attorney Docket No. 38187-2551) filed April 19, 2002.

Figure 3 illustrates the operation of a feedback loop using a processor 60. The processor 60 stores profiles 62 in non-volatile memory. A user inputs information 64 about the desired circumstances or parameters for a lancing event. The processor 60 selects a driver profile 62 from a set of alternative driver profiles that have been preprogrammed in the processor 60 based on typical or desired tissue penetration device performance determined through testing at the factory or as programmed in by the operator. The processor 60 may customize by either scaling or modifying the profile based on additional user input information 64. Once the processor has chosen and customized the profile, the processor 60 is ready to modulate the power from the power supply 66 to the penetrating member driver 68 through an amplifier 70. The processor 60 may measure the location of the penetrating member 72 using a position sensing mechanism 74 through an analog to digital converter 76 linear encoder or other such transducer. Examples of position sensing mechanisms have been described in the embodiments above and may be found in the specification for commonly assigned, copending U.S. Patent Application Ser. No. 10/127,395, (Attorney Docket No. 38187-2551) filed April 19, 2002,

The processor 60 calculates the movement of the penetrating member by comparing the actual profile of the penetrating member to the predetermined profile. The processor 60 modulates the power to the penetrating member driver 68 through a signal generator 78, which may control the amplifier 70 so that the actual velocity profile of the penetrating member does not exceed the predetermined profile by more than a preset error limit. The error limit is the accuracy in the control of the penetrating member.

After the lancing event, the processor 60 can allow the user to rank the results of the lancing event. The processor 60 stores these results and constructs a database 80 for the individual user. Using the database 79, the processor 60 calculates the profile traits such as degree of painlessness, success rate, and blood volume for various profiles 62 depending on user input information 64 to optimize the profile to the individual user for subsequent lancing cycles. These profile traits depend on the characteristic phases of penetrating member advancement and retraction. The processor 60 uses these calculations to optimize profiles 62 for each user. In addition to user input information 64, an internal clock allows storage in the database 79 of information such as the time of day to generate a time stamp for the lancing event and the time between lancing events to anticipate the user's diurnal needs. The database stores information and statistics for each user and each profile that particular user uses.

In addition to varying the profiles, the processor 60 can be used to calculate the appropriate penetrating member diameter and geometry suitable to realize the blood volume required by the user. For example, if the user requires about 1-5 microliter volume of blood, the processor 60 may select a 200 micron diameter penetrating member to achieve these results. For each class of penetrating member, both diameter and penetrating member tip geometry, is stored in the processor 60 to correspond with upper and lower limits of attainable blood volume based on the predetermined displacement and velocity profiles.

The lancing device is capable of prompting the user for information at the beginning and the end of the lancing event to more adequately suit the user. The goal is to either change to a different profile or modify an existing profile. Once the profile is set, the force driving the penetrating member is varied during advancement and retraction to follow the profile. The method of lancing using the lancing device comprises selecting a profile, lancing according to the selected profile, determining lancing profile traits for each characteristic phase of the lancing cycle, and optimizing profile traits for subsequent lancing events.

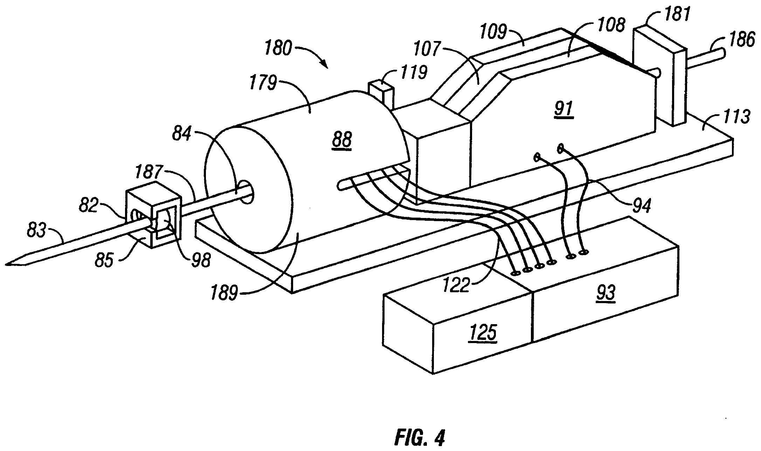

Figure 4 illustrates an embodiment of a tissue penetration device, more specifically, a lancing device 80 that includes a controllable driver 179 coupled to a tissue penetration element. The lancing device 80 has a proximal end 81 and a distal end 82. At the distal end 82 is the tissue penetration element in the form of a penetrating member 83, which is coupled to an elongate coupler shaft 84 by a drive coupler 85. The elongate coupler shaft 84 has a proximal end 86 and a distal end 87. A driver coil pack 88 is disposed about the elongate coupler shaft 84 proximal of the penetrating member 83. A position sensor 91 is disposed about a proximal portion 92 of the elongate coupler shaft 84 and an electrical conductor 94 electrically couples a processor 93 to the position sensor 91. The elongate coupler shaft 84 driven by the driver coil pack 88 controlled by the position sensor 91 and processor 93 form the controllable driver, specifically, a controllable electromagnetic driver.

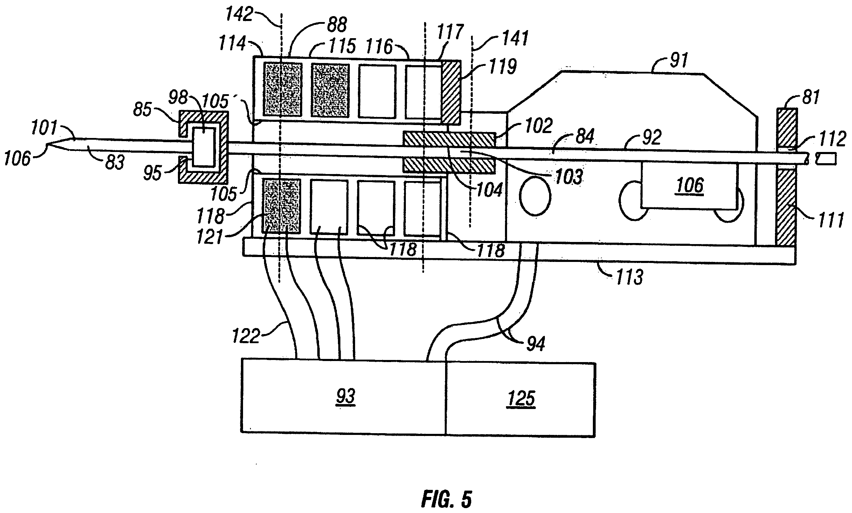

Referring to Figure 5, the lancing device 80 can be seen in more detail, in partial longitudinal section. The penetrating member 83 has a proximal end 95 and a distal end 96 with a sharpened point at the distal end 96 of the penetrating member 83 and a drive head 98 disposed at the proximal end 95 of the penetrating member 83. A penetrating member shaft 201 is disposed between the drive head 98 and the sharpened point 97. The penetrating member shaft 201 may be comprised of stainless steel, or any other suitable material or alloy and have a transverse dimension of about 0.1 to about 0.4 mm. The penetrating member shaft may have a length of about 3 mm to about 50 mm, specifically, about 15 mm to about 20 mm. The drive head 98 of the penetrating member 83 is an enlarged portion having a transverse dimension greater than a transverse dimension of the penetrating member shaft 201 distal of the drive head 98. This configuration allows the drive head 98 to be mechanically captured by the drive coupler 85. The drive head 98 may have a transverse dimension of about 0.5 to about 2 mm.

A magnetic member 102 is secured to the elongate coupler shaft 84 proximal of the drive coupler 85 on a distal portion 203 of the elongate coupler shaft 84. The magnetic member 102 is a substantially cylindrical piece of magnetic material having an axial lumen 204 extending the length of the magnetic member 102. The magnetic member 102 has an outer transverse dimension that allows the magnetic member 102 to slide easily within an axial lumen 105 of a low friction, possibly lubricious, polymer guide tube 105' disposed within the driver coil pack 88. The magnetic member 102 may have an outer transverse dimension of about 1.0 to about 5.0 mm, specifically, about 2.3 to about 2.5 mm. The magnetic member 102 may have a length of about 3.0 to about 5.0 mm, specifically, about 4.7 to about 4.9 mm. The magnetic member 102 can be made from a variety of magnetic materials including ferrous metals such as ferrous steel, iron, ferrite, or the like. The magnetic member 102 may be secured to the distal portion 203 of the elongate coupler shaft 84 by a variety of methods including adhesive or epoxy bonding, welding, crimping or any other suitable method.

Proximal of the magnetic member 102, an optical encoder flag 206 is secured to the elongate coupler shaft 84. The optical encoder flag 206 is configured to move within a slot 107 in the position sensor 91. The slot 107 of the position sensor 91 is formed between a first body portion 108 and a second body portion 109 of the position sensor 91. The slot 107 may have separation width of about 1.5 to about 2.0 mm. The optical encoder flag 206 can have a length of about 14 to about 18 mm, a width of about 3 to about 5 mm and a thickness of about 0.04 to about 0.06 mm.

The optical encoder flag 206 interacts with various optical beams generated by LEDs disposed on or in the position sensor body portions 108 and 109 in a predetermined manner. The interaction of the optical beams generated by the LEDs of the position sensor 91 generates a signal that indicates the longitudinal position of the optical flag 206 relative to the position sensor 91 with a substantially high degree of resolution. The resolution of the position sensor 91 may be about 200 to about 400 cycles per inch, specifically, about 350 to about 370 cycles per inch. The position sensor 91 may have a speed response time (position/time resolution) of 0 to about 120,000 Hz, where one dark and light stripe of the flag constitutes one Hertz, or cycle per second. The position of the optical encoder flag 206 relative to the magnetic member 102, driver coil pack 88 and position sensor 91 is such that the optical encoder 91 can provide precise positional information about the penetrating member 83 over the entire length of the penetrating member's power stroke.

An optical encoder that is suitable for the position sensor 91 is a linear optical incremental encoder, model HEDS 9200, manufactured by Agilent Technologies. The model HEDS 9200 may have a length of about 20 to about 30 mm, a width of about 8 to about 12 mm, and a height of about 9 to about 11 mm. Although the position sensor 91 illustrated is a linear optical incremental encoder, other suitable position sensor embodiments could be used, provided they posses the requisite positional resolution and time response. The HEDS 9200 is a two channel device where the channels are 90 degrees out of phase with each other. This results in a resolution of four times the basic cycle of the flag. These quadrature outputs make it possible for the processor to determine the direction of penetrating member travel. Other suitable position sensors include capacitive encoders, analog reflective sensors, such as the reflective position sensor discussed above, and the like.

A coupler shaft guide 111 is disposed towards the proximal end 81 of the lancing device 80. The guide 111 has a guide lumen 112 disposed in the guide 111 to slidingly accept the proximal portion 92 of the elongate coupler shaft 84. The guide 111 keeps the elongate coupler shaft 84 centered horizontally and vertically in the slot 102 of the optical encoder 91.

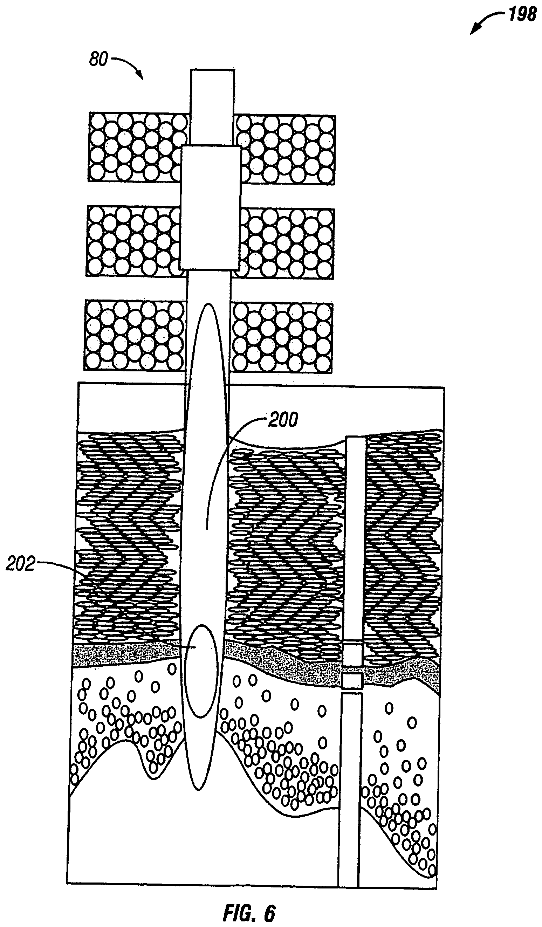

Referring now to Figure 6, one embodiment for a device for fluid sampling is shown. As seen, a patent needle device 198 for accurately positioning a patent needle 200 within the skin layer is shown. The needle 200 has an opening 202 for receiving fluid from the patient.

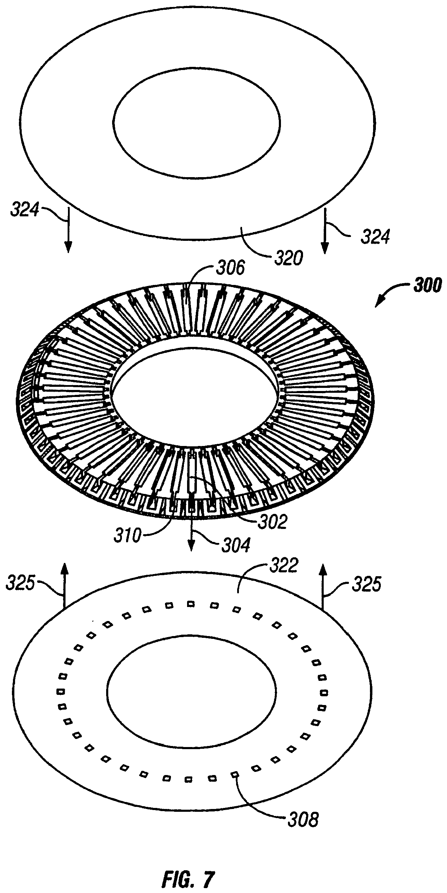

Referring now to Figure 7, a still further embodiment of a cartridge will be described. Figure 7 shows one embodiment of a cartridge 300 which may be removably inserted into an apparatus for driving penetrating members to pierce skin or tissue. The cartridge 300 has a plurality of penetrating members 302 that may be individually or otherwise selectively actuated so that the penetrating members 302 may extend outward from the cartridge, as indicated by arrow 304, to penetrate tissue. In the present embodiment, the cartridge 300 may be based on a flat disc with a number of penetrating members such as, but in no way limited to, (25,50, 75, 100, ...) arranged radially on the disc or cartridge 800.

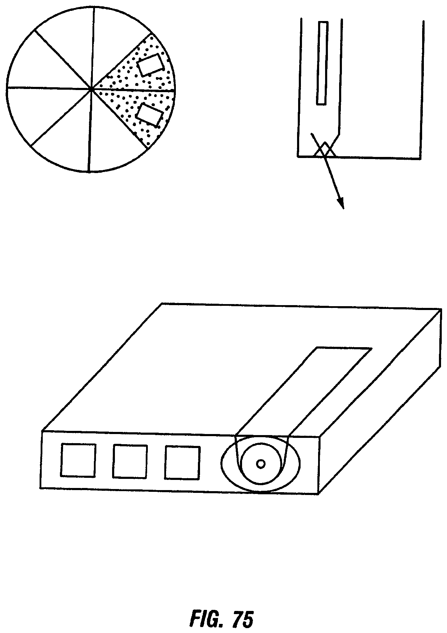

Each penetrating member 302 may be contained in a cavity 306 in the cartridge 300 with the penetrating member's sharpened end facing radially outward and may be in the same plane as that of the cartridge. The cavity 306 may be molded, pressed, forged, or otherwise formed in the cartridge. Although not limited in this manner, the ends of the cavities 306 may be divided into individual fingers (such as one for each cavity) on the outer periphery of the disc. The particular shape of each cavity 306 may be designed to suit the size or shape of the penetrating member therein or the amount of space desired for placement of the analyte detecting members 808. For example and not limitation, the cavity 306 may have a V-shaped cross-section, a U-shaped cross- section, C-shaped cross-section, a multi-level cross section or the other cross-sections. The opening 810 through which a penetrating member 302 may exit to penetrate tissue may also have
a variety of shapes, such as but not limited to, a circular opening, a square or rectangular opening, a U-shaped opening, a narrow opening that only allows the penetrating member to pass, an opening with more clearance on the sides, a slit, a configuration as shown in Figure 75, or the other shapes.

In this embodiment, after actuation, the penetrating member 302 is returned into the cartridge and may be held within the cartridge 300 in a manner so that it is not able to be used again. By way of example and not limitation, a used penetrating member may be returned into the cartridge and held by the launcher in position until the next lancing event. At the time of the next lancing, the launcher may disengage the used penetrating member with the cartridge 300 turned or indexed to the next clean penetrating member such that the cavity holding the used penetrating member is position so that it is not accessible to the user (i.e. turn away from a penetrating member exit opening). In some embodiments, the tip of a used penetrating member may be driven into a protective stop that hold the penetrating member in place after use. The cartridge 300 is replaceable with a new cartridge 300 once all the penetrating members have been used or at such other time or condition as deemed desirable by the user.

Referring still to the embodiment in Figure 7, the cartridge 300 may provide sterile environments for penetrating members via seals, foils, covers, polymeric, or similar materials used to seal the cavities and provide enclosed areas for the penetrating members to rest in. In the present embodiment, a foil or seal layer 320 is applied to one surface of the cartridge 300. The seal layer 320 may be made of a variety of materials such as a metallic foil or other seal materials and may be of a tensile strength and other quality that may provide a sealed, sterile environment until the seal layer 320 is penetrate by a suitable or penetrating device providing a preselected or selected amount of force to open the sealed, sterile environment. Each cavity 306 may be individually sealed with a layer 320 in a manner such that the opening of one cavity does not interfere with the sterility in an adjacent or other cavity in the cartridge 800. As seen in the embodiment of Figure 7, the seal layer 320 may be a planar material that is adhered to a top surface of the cartridge 800.

Depending on the orientation of the cartridge 300 in the penetrating member driver apparatus, the seal layer 320 may be on the top surface, side surface, bottom surface, or other positioned surface. For ease of illustration and discussion of the embodiment of Figure 7, the layer 320 is placed on a top surface of the cartridge 800. The cavities 306 holding the penetrating members 302 are sealed on by the foil layer 320 and thus create the sterile environments for the penetrating members. The foil layer 320 may seal a plurality of cavities 306 or only a select number of cavities as desired.

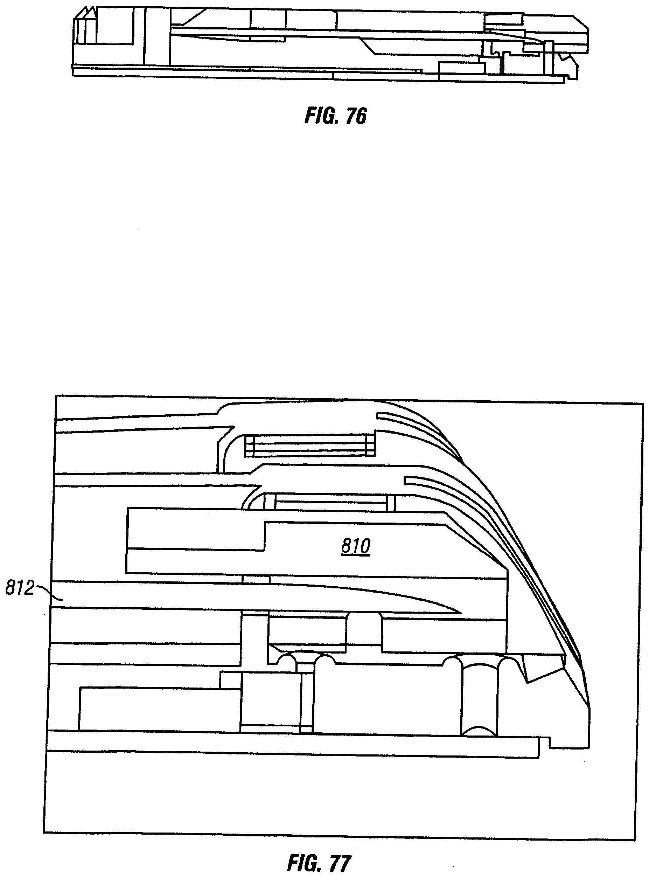

In a still further feature of Figure 7, the cartridge 300 may optionally include a plurality of analyte detecting members 308 on a substrate 322 which may be attached to a bottom surface of the cartridge 300. The substrate may be made of a material such as, but not limited to, a polymer, a foil, or other material suitable for attaching to a cartridge and holding the analyte detecting members 308. As seen in Figure 7, the substrate 322 may hold a plurality of analyte detecting members, such as but not limited to, about 10-50, 50-100, or other combinations of analyte detecting members. This facilitates the assembly and integration of analyte detecting members 308 with cartridge 300. These analyte detecting members 308 may enable an integrated body fluid sampling system where the penetrating members 302 create a wound tract in a target tissue, which expresses body fluid that flows into the cartridge for analyte detection by at least one of the analyte detecting members 308. The substrate 322 may contain any number of analyte detecting members 308 suitable for detecting analytes in cartridge having a plurality of cavities 306. In one embodiment, many analyte detecting members 308 may be printed onto a single substrate 322 which is then adhered to the cartridge to facilitate manufacturing and simplify assembly. The analyte detecting members 308 may be electrochemical in nature. The analyte detecting members 308 may further contain enzymes, dyes, or other detectors which react when exposed to the desired analyte. Additionally, the analyte detecting members 308 may comprise of clear optical windows that allow light to pass into the body fluid for analyte analysis. The number, location, and type of analyte detecting member 308 may be varied as desired, based in part on the design of the cartridge, number of analytes to be measured, the need for analyte detecting member calibration, and the sensitivity of the analyte detecting members. If the cartridge 300 uses an analyte detecting member arrangement where the analyte detecting members are on a substrate attached to the bottom of the cartridge, there may be through holes (as shown in Figure 76), wicking elements, capillary tube or other devices on the cartridge 300 to allow body fluid to flow from the cartridge to the analyte detecting members 308 for analysis. In other configurations, the analyte detecting members 308 may be printed, formed, or otherwise located directly in the cavities housing the penetrating members 302 or areas on the cartridge surface that receive blood after lancing.

The use of the seal layer 320 and substrate or analyte detecting member layer 322 may facilitate the manufacture of these cartridges 10. For example, a single seal layer 320 may be adhered, attached, or otherwise coupled to the cartridge 300 as indicated by arrows 324 to seal many of the cavities 306 at one time. A sheet 322 of analyte detecting members may also be adhered, attached, or otherwise coupled to the cartridge 300 as indicated by arrows 325 to provide many analyte detecting members on the cartridge at one time. During manufacturing of one embodiment the cartridge 300 may be loaded with penetrating members 302, sealed with layer 320 and a temporary layer (not shown) on the bottom where substrate 322 would later go, to provide a sealed environment for the penetrating members. This assembly with the temporary bottom layer is then taken to be sterilized. After sterilization, the assembly is taken to a clean room (or it may already be in a clear room or equivalent environment) where the temporary bottom layer is removed and the substrate 322 with analyte detecting members is coupled to the cartridge as shown in Figure 7. This process allows for the sterile assembly of the cartridge with the penetrating members 302 using processes and/or temperatures that may degrade the accuracy or functionality of the analyte detecting members on substrate 322. As a nonlimiting example, the entire cartridge 300 may then be placed in a further sealed container such as a pouch, bag, plastic molded container, etc...to facilitate contact, improve ruggedness, and/or allow for easier handling.

In some embodiments, more than one seal layer 320 may be used to seal the cavities 306. As examples of some embodiments, multiple layers may be placed over each cavity 306, half or some selected portion of the cavities may be sealed with one layer with the other half or selected portion of the cavities sealed with another sheet or layer, different shaped cavities may use different seal layer, or the like. The seal layer 320 may have different physical properties, such as those covering the penetrating members 302 near the end of the cartridge may have a different color such as red to indicate to the user (if visually inspectable) that the user is down to say 10, 5, or other number of penetrating members before the cartridge should be changed out.

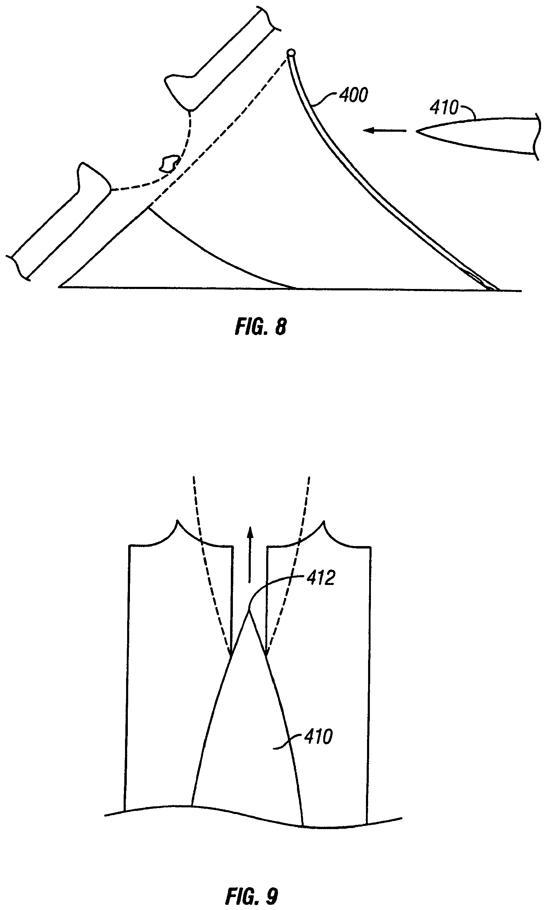

Referring now to Figures 8 and 9, one embodiment is shown. This embodiment provides a solution to a problem, which concerns the possible inability to guarantee a stable blood volume from a finger penetrating member wound to a detecting member port located on a disposable cartridge. The problem might be due to shallowness of the penetrating member penetration depth, skin surface tension issues, or the patient's vascular conditions resulting in the invariability of the blood volume from the penetrating member wound. Otherwise known as the patient's inability to produce an adequate blood droplet shape and size. There have been other stated solutions such as the delivery of the penetrating member to the finger with a deeper penetration depth or a programmed controlled "penetrating member-in-the-finger" dwell time to sustain the size of the wound, which allows more blood to be produced from the wound. However, each of these might possibly result in a compromise on the degree of pain or sensation felt by the patient.

In one embodiment, a concept of a "gang plank" for the blood to travel directly from the wound to the analyte detecting member port on the cartridge is provided. The device may use a cantilevered section that is movable. Thus the volume of blood produced at the wound site irregardless of its droplet geometry can be completely transported to the analyte detecting member. The disclosure relates to the problems in blood volume invariability during the post penetrating member wound generation and blood droplet sampling. The technical field relates to blood sampling and transport to diagnostic instrumentation sensing devices. The battle is how to get the gang plank in place. In one embodiment, the tip of the penetrating member will not contact the plank. The plank may have a slot and the slot allows the tip to protrude through without being touched. The material of the plank may be soft and pliable. In one embodiment, the penetrating member comes through and the portion will fall down. Droplet forms, touches, and wicks it. It can be capillary or surface tension. The disclosure solves how do you bridge the gap between the sampling area. All of this will be described in further detail below.

Referring now to Figure 8, one embodiment produces a concept of a cantilevered portion 400 for the blood to travel directly from the wound W to the analyte detecting member port on the cartridge. The portion 400 may be flexible and movable from a first configuration to a second configuration. Thus the volume of blood produced at the wound site W irregardless of its droplet geometry can be completely transported to the analyte detecting member which receive fluid from the portion 400.

The problem concerns the possible inability to guaranteed blood flowing from the finger wound to the analyte detecting member port located on the disposable cartridge. The problem might be the invariability of the blood volume from the penetrating member wound. Otherwise known as the shape and size of the droplet. There have been stated solutions such as the delivery of the penetrating member to the finger with a deeper penetration depth or a programmed controlled "penetrating member-in-the-finger" dwell time to sustain the size of the wound, which allows more blood to be produced from the wound. However, each might possibly result in a compromise on the degree of pain or sensation felt by the patient.

In one embodiment, the disclosure provides for the blood or other sample fluid to travel directly from the wound W to the analyte detecting member port on the cartridge. In one embodiment, it was concluded that the penetrating member 410 or even the foil breaker might be used as the device to launch the gangplank 400. After all, in one embodiment the penetrating member 410 and foil breaker are already in existence on the instrument and their simple motions in either breaking the foil or launching the penetrating member might also be used in launching the gangplank 400.

As seen in Figure 8, the gangplank 400 may be a molded break away hinged flap originally connecting between the upper and lower halves of the port directly in the way of the penetrating member 410. The gangplank 400 may have a breakaway connection 404 that holds the gangplank 400 in position prior to use. If the penetrating member were to be launched, the forward momentum generated by the penetrating member impacting the gangplank 400 would force the gangplank 400out of the way. This is provided that the gang plank is correctly hinged on the bottom and very thinly connected on the top as to allow the force of the impact to tear away the top connections loose and allow the gang plank to swing down vertically with respect to the lower hinges as to lie down flat securely between the wound and the analyte detecting member hole.

What about the sharp penetrating member point? Would the impact of the penetrating member point on the gangplank 400 damage the penetrating member point integrity? Consider that the penetrating member is not directly impacting the gangplank 400, but merely a glancing blow and the servo controlled launching mechanism can be programmed to provide the adequate forward momentum as to gently push the gangplank 400 out of the way. In one embodiment, the servo control methods being used to gently place the penetrating member tip against the skin might also be used to gently force the gangplank 400 down upon the skin at the proximal site of the impending penetrating member wound.

Referring now to Figure 9, consider if the gangplank 400 were to be slight hyperbolic slotted (shown in phantom) and as to provide a small opening 412 to allow the penetrating member tip to be unobstructed but enough for the remaining secondary and primary cutting edges to force the gang plank 400 down. Also consider that the upright surface on the gang plank between the wound and analyte detecting member hole may be treated as to be hydrophobic on the side edges and hydrophilic on the center portions as to provide a preferential flow channel path for blood to flow through capillary action to traverse between the wound and analyte detecting member hole on the cartridge.

In one embodiment, fabrication may involve the use of the plastic molding processes, except that there will be a small plastic gangplank 400 hanging across the penetrating member path. As mentioned earlier, the gangplank 400 by design will be made in one embodiment to snap loose on top and to hinge downward on the bottom. It should be noted that the downward sweeping motion of the foil breaker or the outward motion of the penetrating member could contribute to the single action of placing the gangplank 400 in the location between the blood droplet produced by the subsequent lancing action and the cartridge analyte detecting member hole. Also if it were the motion of the foil breaker to force the gangplank 400 in place, the clearance slot on the gangplank 400 for the penetrating member clearance might not be needed at all. However, if it were to be guarantee that the gangplank 400 is launched and placed properly, then the use of either the foil breaker or the penetrating member might be desired. But in either cases, both motions and the plastic structural material is already in place and can be implemented by design with either no added or very little increase in parts and cost.

In one embodiment, a small hump or bump in the plastic in front of the beveled cartridge entrance will have to implemented as both penetrating member guide or a door stop for the gangplank 400 to allow proper positioning in front of the blood droplet resulting from the penetrating member wound.

In one embodiment, the penetrating member tip does not touch the plank. It is a shaped opening. The penetrating members are going to come out and the orientation is not quite known. The sides or backs will touch the gang plank. The plank, in this embodiment, may be designed so that it will not spring back up.

In one embodiment, the distance between the finger and the plank is about 100-300 microns. To allow formation of the droplet to an unstable point so it can be attracted. In one embodiment, the disclosure provides a movable object that is lowered by the lancing motion and the object is within 100-300 microns of the finger such that the droplet will be through natural forces, attracted onto the plank (hydrophillic). And the capillary action will allow a sizable quantity fluid.

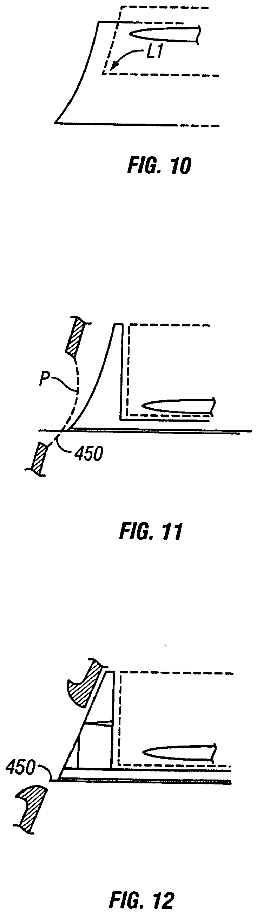

Referring now to Figures 10 through 18, the technical field of this next embodiment relates to the measurement of finger forces placed against an aperture annulus. It also calls for the design and implementation of a thick film strain gage on a flexible polymer circuit material. When a finger is sticking through a an opening on a lancing device (finger pouching or protruding), how does the user know how much force a user should push forward with. Neural distraction prevents a user from discriminating if the finger pouch provides enough force. How much force does a user place? It is generally too expensive to use force gauges that are known.

In one embodiment, the disclosure uses screen printed carbon lines as force gauges (in a resistive bridge). Resistors can be used to create a wheatstone (width is fixed, length is variable). The fulcrum point causes stress (Delta L), the slight change in resistance indicates that there is contact. In one embodiment, the light may indicate that finger is in contact or it may be used to automatically fire the penetrating member. The disclosure provides a smart, disposable front end that can indicate if tissue has made contact. It may be made of resistive analyte detecting members that deform that changes resistance changes and indicates the amount of force. The carbon lines can be placed anywhere, but may be where the fulcrum point will be.

Referring now to Figure 10 and 11, this device detects the presence of an adequate finger pooching P within the defined aperture window. The simple appearance of the finger within the aperture does not guarantee the desired requirements of quantifying the degree of finger pooching. This is needed in order to provide some measurement of either the degree of force the finger is placing upon the front-end aperture or the amount of finger surface tissue protruding through the aperture. Another desire of this disclosure is its implementation with minimal costs and components on the disposable cartridge.

Figures 10-13 depict several variations of the penetrating member and disposable blood sampling cartridges. The device is shown along with the depiction of the top view showing the flexible smart squeegee mechanism with strain gages. In another drawing, the presence and absence of an adequate finger protrusion in the front-end annular annulus is shown along with the simple electronics.

This device requires the use of the existing lower half of the disposable cartridge which may be similar to that as seen in Figure 7. This lower half contains the deposited conductive electrical traces and the chemical analyte detecting members. The device calls for
the design of a lower lip that is thin and flexible and protruding close to the aperture window. This protrusion will resemble a very flexible squeegee like structure 450. However, it is not the intention of this squeegee like structure to transport or come in contact with the blood. The intent of this flexible protrusion 450 will be to signal the user via the instrument that an adequate amount of finger skin pooching has occurred and an acceptable distance between the finger and the disposable cartridge has been reached.

In one embodiment, the intent of this structure 450 is to develop a user friendly and simple analyte detecting member to indicate when the user has applied enough force to allow adequate pooching within the aperture window as to guarantee the eventual outcome of sufficient blood transport conditions from the fingertip to the disposable cartridge. The flexible squeegee 450 may be made of a thin portion of the lower plastic rim belonging to the chemistry and conducting traces for the analyte detecting members. In one embodiment, it may contain two wires from the electronics of which can be picked up from the instrument with commutator like contacts. The thin film deposited lines will go off to the squeegee like flex circuit.

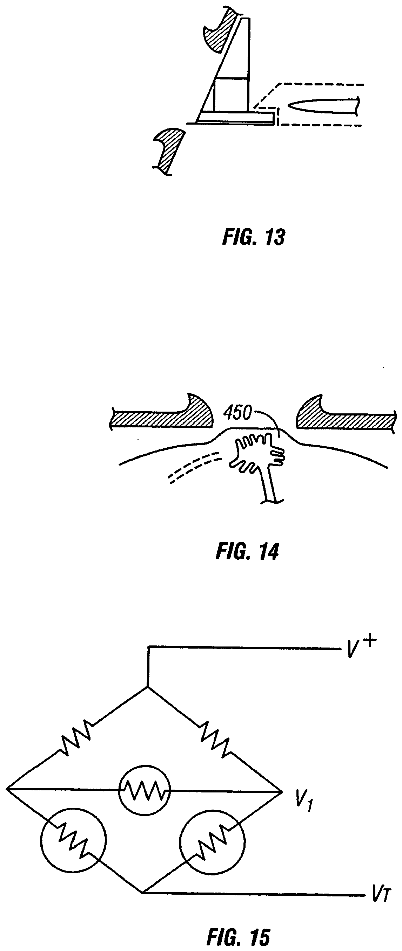

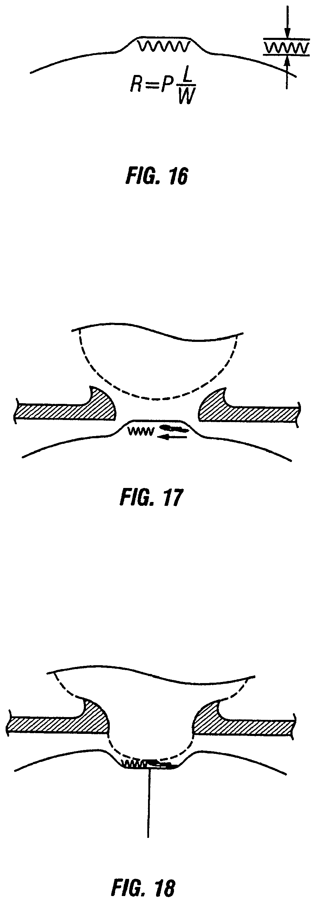

Referring now to Figure 14, when the user applies their finger into the aperture, they will push up against the squeegee like structure. When adequate enough protrusion comes thru, they will bend the squeegee 450 and vary the resistance on the electrical circuit. The circuit might be part of a Wheatstone bridge like circuit on the either the flex circuit or within the instrument, as seen in Figures 14 and 15. Upon meeting the correct electronic parameters, the user will receive a signal. This signal will tell the user that they had pushed hard and far enough, as seen in Figure 16. In some embodiments, maintaining the pressure, the user will then fire the penetrating member launcher. Figure 18 shows the pouching and moving of the strain gauge.

The flexing of the squeegee will allow the protruding tip to move out of the way and thus will interfere with neither the launcher tip nor the blood droplet.

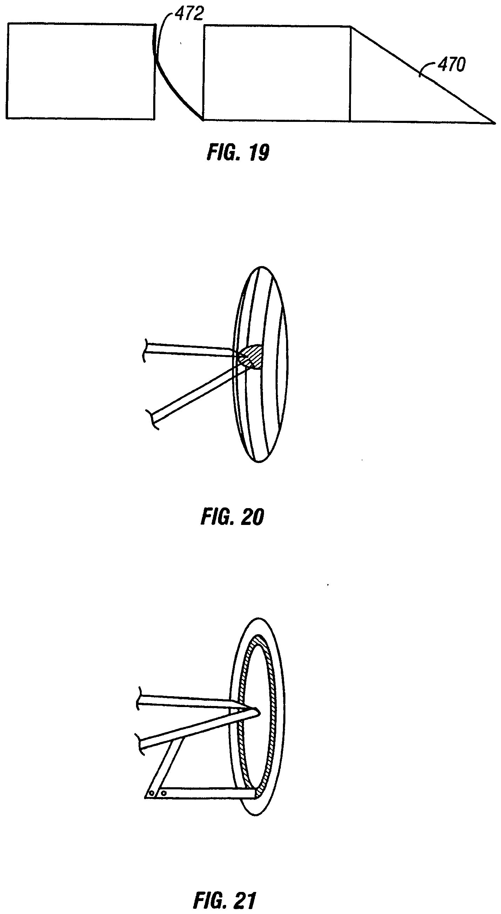

Referring now to Figures 19 to 20, the disclosure relates to the problems in blood volume invariability during the post penetrating member wound generation and blood droplet sampling. The technical field relates to blood sampling and transport to diagnostic instrumentation sensing devices.

In one embodiment, the disclosure provides a solution to a problem, which concerns the possible inability to guaranteed a stable blood volume from a finger penetrating member wound to a analyte detecting member port located on a disposable cartridge. The problem might be due to shallowness of the penetrating member penetration depth, skin surface tension issues, or the patient's vascular conditions resulting in the invariability in achieving an adequate blood droplet shape and size. There have been other stated solutions such as the delivery of the penetrating member to the finger with a deeper penetration depth or a control method to increase the amount of blood to be produced from the wound.

In one embodiment, this disclosure produces a concept to allow the control of the distance between the skin and the pickup of the analyte detecting member. Given a desired size blood droplet, the blood would travel directly from the wound into the analyte detecting member port on the cartridge.

Referring now to Figure 19, one embodiment of a penetrating member is shown. The penetrating member 470 includes a leaf spring 472. The problem concerns the possible inability to guarantee blood flowing from the finger penetrating member wound to the analyte detecting member port located on the disposable cartridge. The problem might be the invariability of the blood volume from the penetrating member wound. Given that a certain volume of blood is required to fill the analyte detecting member, and that the droplet forms best when it isn't in contact with any other surface. It is desirable to adjust the standoff distance to the point where the droplet is of a potential to fill the analyte detecting member when the droplet hits the pickup surface for the analyte detecting member.

In one embodiment, if a small o-ring located inside of the devices fixed aperture is attached to the pickup and allowed to float, the offset uncertainty that is incurred by the amount of doming of the skin into the aperture can be corrected for.

As shown in the embodiment of Figure 19, the suspension of the pickup could be accomplished with a leaf spring that connects the penetrating member cartridge to the analyte detecting member cartridge. In this leaf spring concept, the pickup surface is the beveled surface on the right. A spring 472 suspends the analyte detecting member cartridge on the right to the penetrating member cartridge on the left.

Referring now to Figures 20-22, the initial concept of suspending the blood acquisition pickup with a suspension mechanism is shown. This embodiment produces a concept of a "suspender" for the blood to travel from the wound to the analyte detecting member port on the cartridge when the droplet has reached the optimal size. With an experimentally derived adequate size, the droplet will be assured of possessing a certain energy potential. Upon subsequent contact with the pickup of the sampling geometry, the volume of blood produced at the wound site will be assured of adequately filling the analyte detecting member chamber and allow sensing.

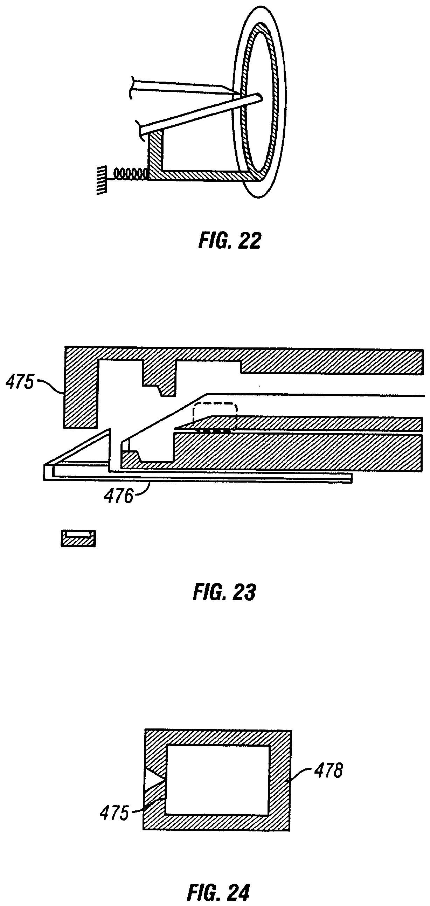

Referring now to Figure 23 and 24, the disclosure provides a punch device 475 that has two portions, one portion for break a sterility barrier over a penetrating member cavity and one for breaking the barrier over analyte sensing cavity. A separate ring or disc 476 may be provided to attach to an existing penetrating member cartridge.

Referring now to Figure 24, a heat seal may be varied so that more of the barrier is coupled to one portion 478 of the cartridge than a front portion 479 to give the portion 478 greater strength and thus control the breaking away of the foil.

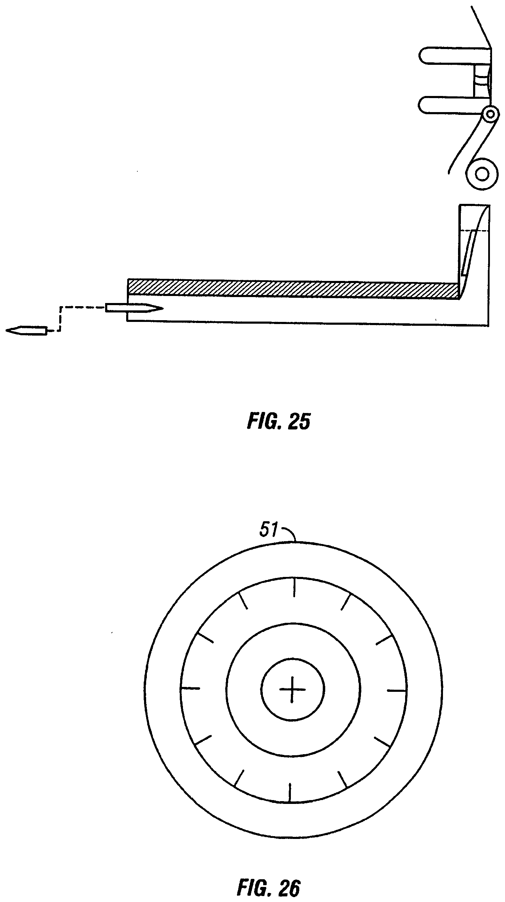

Referring now to Figure 25, one embodiment showing a peeling mechanism to release the sterility barrier is shown.

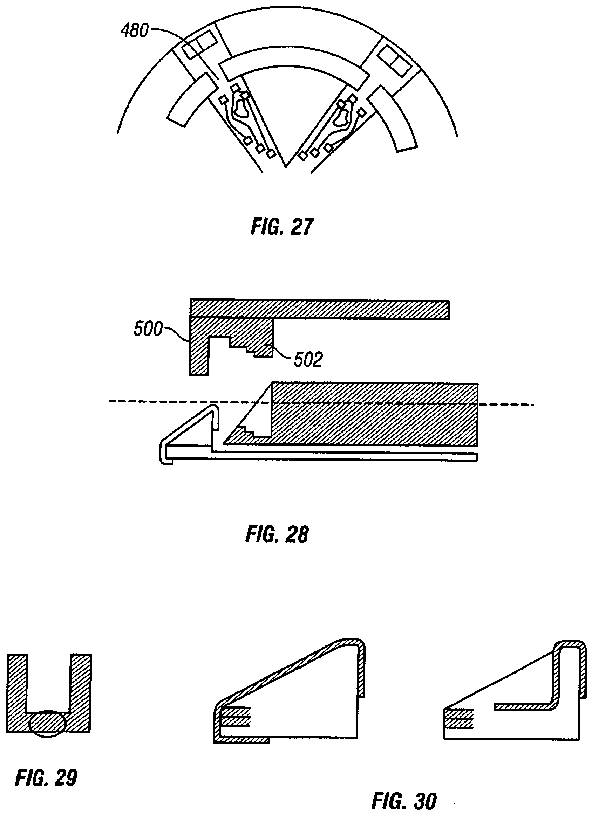

Referring now to Figures 26 and 27, the device is shown with the underside of the cartridge or disc with cavities to facilitate removal from a mold. A stem or extension 480 is provided so that electrode lines can extend across these cavities. In the present embodiments, these are formed on the underside. Some embodiments may have the structures formed on the top side of the cartridge.

Referring now to Figure 28, the disclosure pertains to the technical field of polymer film bonding and adhesion, sterility sealing, environmental sealing, and the removal of such films during physiological measurements.

The disclosure relates to the use of a plastic or mylar-like film to cover the measurement analyte detecting member structure where exposure to the elements is decrement to the life of the analyte detecting member chemistry. The disclosure pertains to both the cover and the mechanism desired to remove the protective film cover without residue particulates from the removed film coming lose as to become contaminants during the measurement and bodily fluid sampling process.

The problem relates to providing a analyte detecting member structure protection from the environment during storage. Equally as important is its removal during the sample preparation process. This disclosure introduces both the use of a protective film and the device to remove the respective film.

As seen in Figure 28, the top sketch shows the addition of a film removal punch 500 to the penetrating member protective foil punch 502. The arm extension shown on the sketch provides a single downward motion as illustrated in the subsequent sketches. The downward motion draws the film away from the sampling port like that of a window shade being drawn.

Referring now to Figures 29 to 30, the current foil seal and opening utilizes a stationary knife or staircase stamping structure that forces the foil into a corner below its former self. The opening system has neither forward nor backward motion. It goes only upward to break the foil seal and forced it into the corner pocket and a downward to get out of the way prior to the indexing motion of the cartridge wheel. The solution was to use the existing opening system. However, the material used in this embodiment will not be foil but rather a plastic film, which will be selectively heat-sealed in the back locations to provide a permanent seal and anchor. The frontal and bottom locations may be weakly heat sealed to provide breakpoints along the bottom and front such as the film will be allowed to collapse downward and inward. Thus pulling away from the analyte detecting member entry areas as shown in Figure 30.

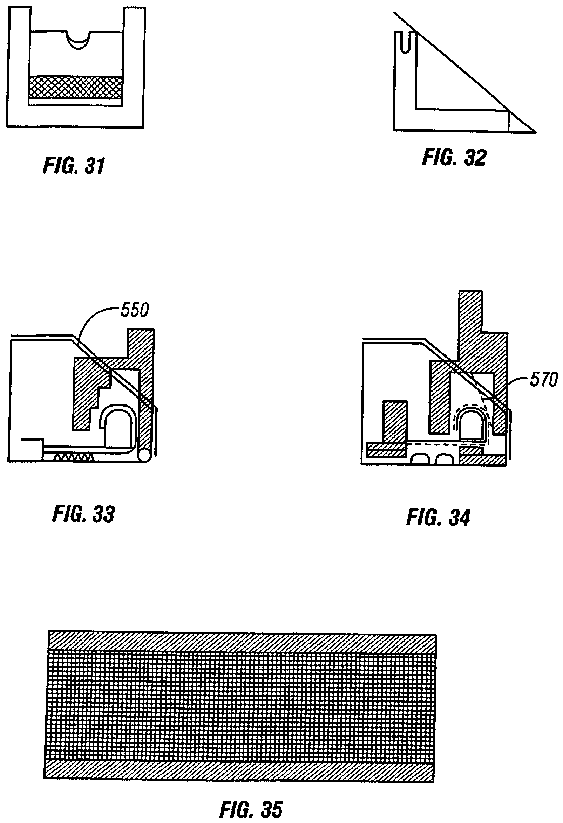

Referring now to Figure 31, the disclosure relates to the structure sealing that will allow the sealing of both a penetrating member and analyte detecting member structure. The technical field relates to the methods of foil sealing and adhesion to the plastic cartridge as to allow protection, sterility, and isolation of both a penetrating member and electrochemical analyte detecting member structure. The present invention addresses sealing sensing device and penetrating member. Sterlization may harm the analyte detecting member. The disclosure provides a punching mechanism to remove the foil covering the analyte detecting member. The punch moves down to remove the foil from the front face area. A beveled area or wedge 570 may be used to clear away the foil that is partially split and shoves it out of the way. In one embodiment, the disclosure may allow a double cut using a straight vertical motion of the punch. This allows for analyte detecting member and penetrating member separation.

The teeth structure is there it prevent contact with certain features. This pushes straight down. This compresses the foil to be out of the way. If the devices are scraped, that is less desirable to pieces floating around. This compresses the foil into an area.

In one embodiment, this disclosure allows a structure that accommodates both the existence and protection of bodily fluid sampling penetrating member and physiological electrochemical analyte detecting members on a disposable cartridge structure. This disclosure includes the structure of sealing, method of opening, and method of sampling the bodily fluid.

Referring now to the embodiment of Figure 31, the sketch shows a very similar structure as the current front end and foil opening mechanism. In Figures 32 and 33, the foil 550 wraps over the top of the cartridge and covers both the penetrating member and the analyte detecting member region. In this embodiment, this will allow the foil 550 to be sealed with the edges of the bottom plate thickness per the manufacturing limitation. The analyte detecting member is loaded into the pocket from the bottom. The small cavity between the front face and the penetrating member parked location remains open or partially open. Such as to provide a topside exposure to glucose analyte detecting members per IP limitation. The width of the individual unit remains the same and the angle also remains the same.

As seen in the embodiment of Figures 34-35, the foil 550 wraps over the top of the cartridge and covers both the penetrating member and the analyte detecting member region. This will allow the foil to be sealed with the edges of the bottom plate thickness per the manufacturing limitation. The analyte detecting member is loaded into the pocket from the bottom. In one embodiment, the small cavity between the front face and the penetrating member parked location remains open or partially open. Such as to provide a topside exposure to glucose analyte detecting members per IP limitation. The width of the individual unit remains the same and the angle also remains the same.

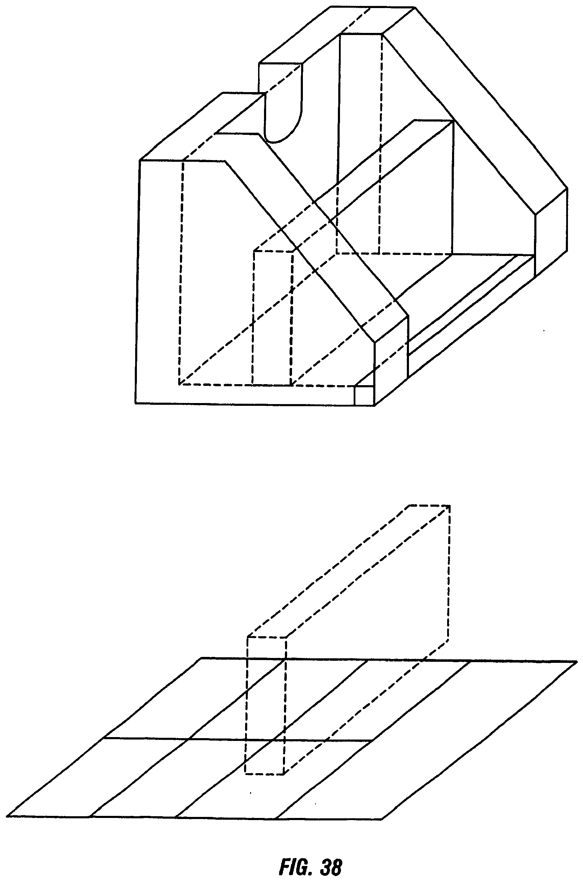

As seen in Figure 38, in some embodiments, there may be a small rising wall 501 that will not exceed the angular coverage of the foil surface. This additional rising wall is between the back penetrating member bearing surface and the base tip, which anchors the foil. Although not limited to the following, this small rising wall may contain a Mylar covered 75 to 100 micron wicking mesh fluidic channel structure. In one embodiment, the Mylar mesh will wrap around the top of the rising wall as to provide an upper anchor for the mesh. The Mylar mesh comes down the rising wall and wraps below to allow the bodily fluid to flow upon the analyte detecting member, which is facing upward.

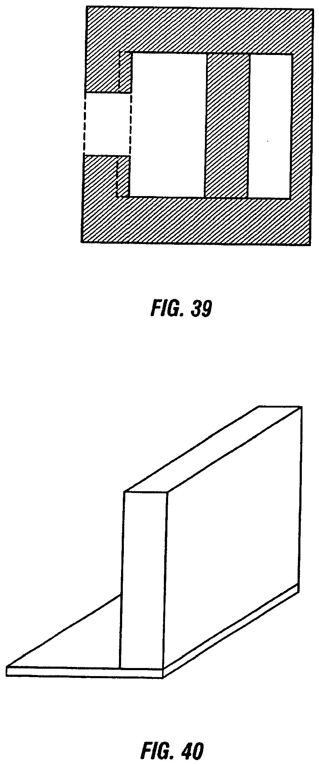

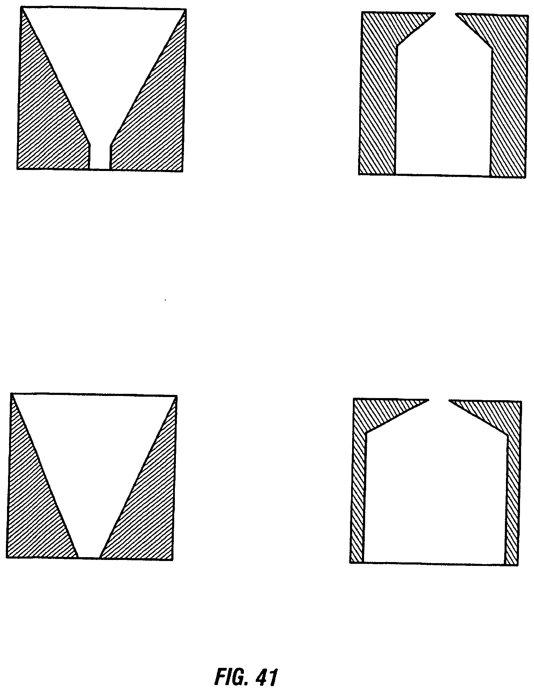

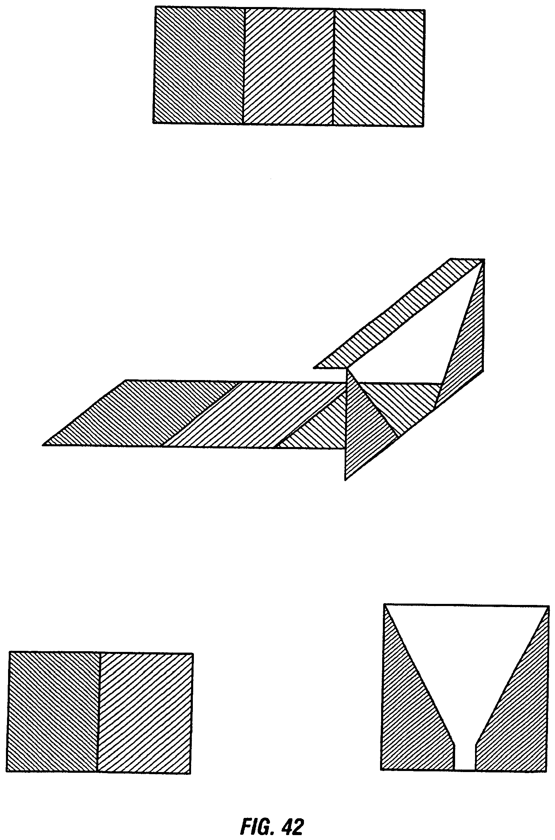

Figures 39 through 43 show additional embodiments or modification that can be made to the device.

The foil opening downward moving arm may have an additional downward moving addition. The original downward arm will possibly need to be shortened back slightly as to provide clearance for the fore mentioned rising mesh supporting wall. The original downward motion of the current foil opener will still break and clear the foil. The pocket for the residual foil will still be at the bottom corner. However, the bottom will be a sheet of Mylar belonging to the Mylar cover mesh. A small portion of the Mylar will be removed as to expose part of the mesh. It is believed that the foil will not interfere with the fluidic flow of the bodily fluids as it travels along the mesh and Mylar surfaces.

The bodily fluid travel will proceed as follow, the Mylar covered mesh structure allows a possible solution to both the lateral and vertical offset problems with respect to the bodily fluid droplet to frontal facial bodily fluid transport structure tolerances. The bodily fluid will make contact with the mesh and travel along the pat of the mesh and down the front face and via the mesh covered Mylar to the glucose analyte detecting members, which are facing upward on the top surface of the cartridge unit parallel to the penetrating member.

In some embodiments, the teeth structure in the punch (as seen in Figure 34) is there it prevent contact with certain features. This pushes straight down. This compresses the foil to be out of the way. If the devices are scraped, that is less desirable to pieces floating around. This compresses the foil into an area.

In one embodiment, the disclosure has allowed the development of the structure to be fabricated in a two-part snap on bottom and top layer to be more economically feasible as to also allow the clearance, sterilization, and mesh assembly.

The foil opening punch will continue to work as it was intended to perform in the downward foil opening motion. However, in some embodiments, it may be shortened in forward length slightly as to allow the penetrating member protecting foil to be opened and packed behind the mesh supporting rising wall. It may also have an additional appendage as to allow opening along the forward front portion of the rising wall to the front tip of the cartridge. In one embodiment, the foil should clear the rising center wall with the mesh. The height of the clearance in this region as depicted by the foil angle will determine the exact height of this rising center wall.

In conclusion, embodiments allow the following:
1). Retention of the current foil and foil opening system.
2). Placement of the glucose analyte detecting member on the top surface parallel to the penetrating member.
3). Use of a vertical mesh wall structure similar to the frontal facial analyte detecting member structure
4). Relaxing of the lateral and vertical offset requirements due to the use of a mesh fluid conduction structure for transport of the droplet to the analyte detecting member structure.
5). A use of the individual cartridge side walls as standoffs to prevent smearing of the droplet due to skin and analyte detecting member or mesh direct contact.

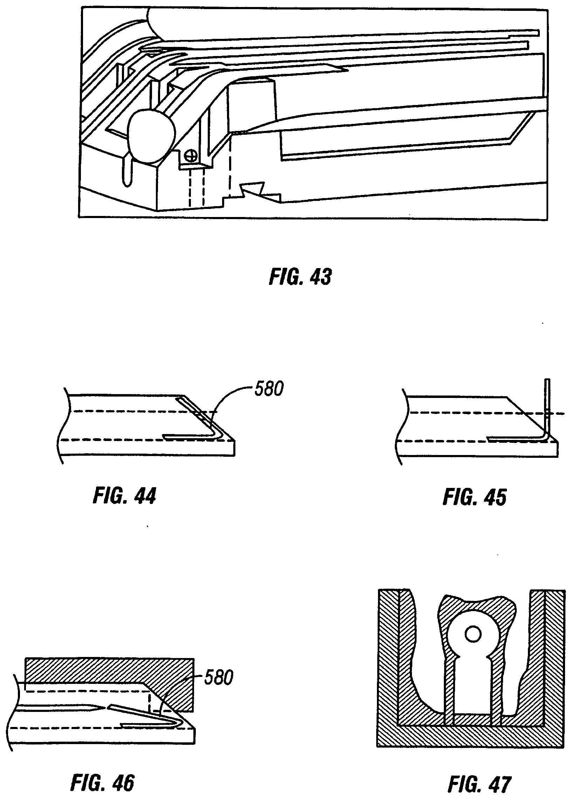

Referring now to Figures 44 to 47, this embodiment relates to using the electronic tissue penetration device to drive a penetrating member into skin to a predetermined depth, and then adapt the front end geometry to consistently gather a spontaneous droplet of blood for analysis. Given that the penetrating member channel is integrated (in some embodiments) with sample inlet, there is a preferred geometry for lancing and a preferred geometry for collection. This concept uses wicking structures to collect and deliver the sample to the analyte detecting member. The disclosure uses the foil seal to package the sample capture structure. When the foil is punched, the sample capture tilt up into position with the laminated wicking material in a desired position for sample fluid collection.

Referring now to Figures 44 and 45, the positioning of the finger near the sample capture structure is desired for the sample capture to capture the blood sample. This device is an assembly that tilts the sample capture structure 580 forward out of the disposable structure substrate after the seal or foil is punched. This is more clearly shown in Figure 45 where the structure 580 is now out of the foil and vertical. This device allows the sterile sealing of the penetrating member and the sample capture structure, the reproducible piercing of the seal to expose the penetrating member and sample capture structure, and the function of the penetrating member sample capture mechanism. Figure 46 shows that during the punch, the structure 580 is pliable and maybe moved downward. Figure 47 shows a front view with the structure 580 positioned as shown in Figure 45, ready to engage tissue for sample capture.

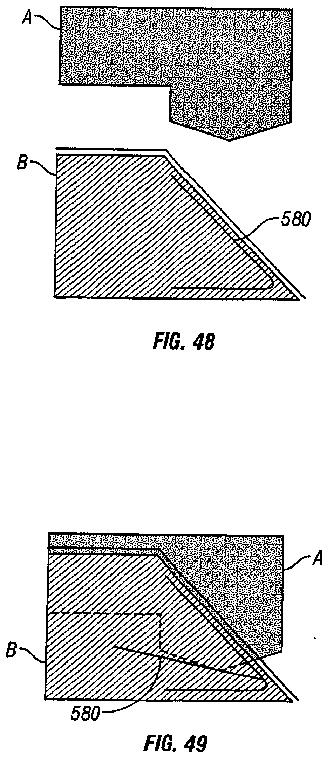

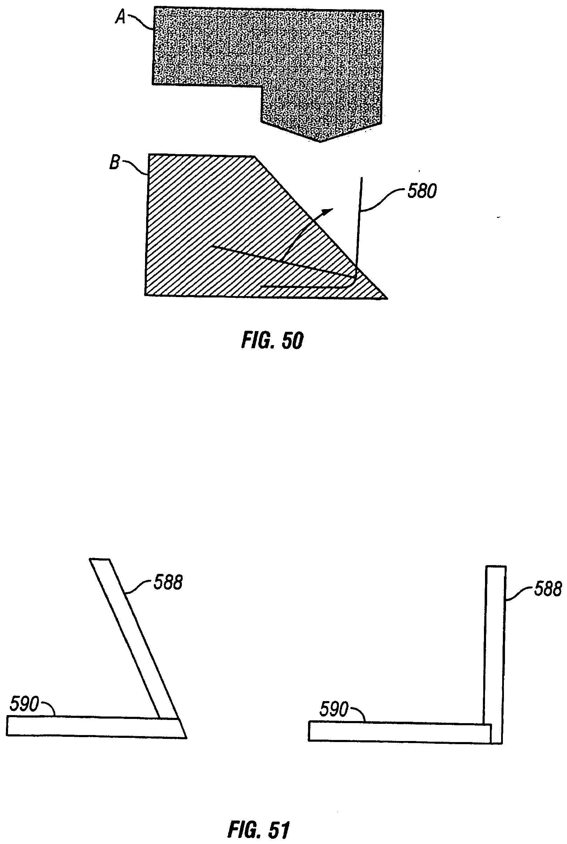

Referring now to Figure 48, one embodiment will be shown in greater detail. In Figure 48, the foil punch (A), the substrate cavity (B), and structure 580 are shown. In Figure 49, the foil punch (A) descends into the substrate cavity (B) to perforate the foil and expose the penetrating member. The sample capture mechanism 580, which is held by the foil, is briefly bent elastically by the punch. As seen in Figure 50, after the punch is raised, the sample capture mechanism 580 is raised. It should be understood that there may be a stop to prevent the mechanism 580 from extending beyond a vertical position.

Referring now to Figure 51, this embodiment relates to a solution to isolating the glucose analyte detecting members from the atmosphere. The technical field relates to material properties of plastics and the ability to define memory or degrees of permanent deformation in plastics. In addition, the technical field of should also include the chemical degradation of glucose oxidase due to its exposure to the atmosphere.

The exposure of the glucose analyte detecting members may create a deviation from the established calibration point and the point of use. The exclusion of the atmosphere from the analyte detecting member is desired. The plastics being used on the cartridge of Figure 7 or the fluid transport structures contain a certain degree of "springiness" or elasticity. This elasticity can be used to our benefit and is similar to plastic tubing being bent over. The bending of the plastic tubing collapses the walls of the tubing onto itself as to form a seal that isolates one end of the tubing from the other. When the tubing 588 is returned to its natural state whether from a 45-degree bend to a 90-degree bend or a 45-degree to a 180-degree plane, the tubing channel will be restored. The fabrication of the tubing structure is performed in the beginning as a planar process. The structure is then die cut when the structure is complete. Prior to the foil sealing process, the planar channel structure (tubing structure) is folded (bent) from its natural position to 45 degrees and sealed within the foiled package. Because the channel structure is bent over, the analyte detecting members are isolated from the atmosphere. When the foil is punctured and opened for penetrating member access, the channel structure is allowed to relax to its normal unbent position, allowing free channel flow between the mesh front end capture structure and the analyte detecting members.

In Figure 51, the drawing shows a left view and a right view of a planar plastic tube structure 588. The bend 590 in the tube allows the passage of fluid from the top to the bottom. Unfortunately, the tube also allows the atmosphere to flow from top down to the surface of the analyte detecting member face. In sense, causing the analyte detecting member to be exposed to the atmosphere and either degrade or deviate from its initial manufacturing calibration point. The right view shows the tubular structure to be unobstructed whereas the left view shows the tube structure to be obstructed by the natural collapsing of the tube inner and outer tube surfaces when the tube is bent.

The plastics being used on the cartridge or the fluid transport structures contain a certain degree of "springiness" or elasticity. This elasticity can be used to our benefit and is similar to plastic tubing being bent over. The bending of the plastic tubing collapses the walls of the tubing onto itself as to form a seal that isolates one end of the tubing from the other.

In one embodiment, the disclosure describes how to seal glucose analyte detecting member from oxygen. In one embodiment if, the tube if cut at the right place can be buckled at the right place. The inner corner buckles in. The 90 degree bend may cause adhesive to slide in. A planar tube may be used by design as a valve.

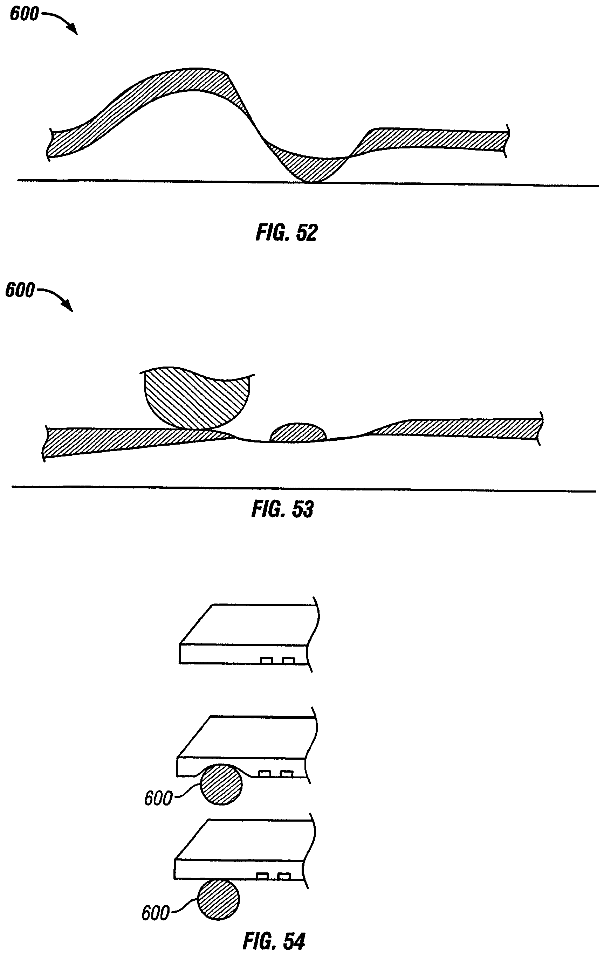

Referring now to Figures 52 and 53, the disclosure is in a technical field that relates to the shaping and flexing of the plastic surface by molding as to cause the natural relaxed state of the plastic planar surface to be formed into a normally closed valve. This pertains to the material properties of plastics as used in the formation of valvular structures. The disclosure relates to concept that utilizes the natural elasticity of the plastic channel and transport structures currently being used. The concept allows a hybrid channel structure comprising of mesh transport and open channel capillary structures. The open area capillary structure is fabricated with a normally closed valve structure. This valve is designed and fabricated in the plastic channel structure of the channel upper or cap layer.

Referring now to Figure 52, this embodiment relates to concept that utilizes the natural elasticity of the plastic channel and transport structures currently being used. The concept allows a hybrid channel structure comprising of mesh transport and open channel capillary structures. The open area capillary structure is fabricated with a normally closed valve structure. This valve is designed and fabricated in the plastic channel structure of the channel upper or cap layer. The plastic channel valve is opened when the respective penetrating member/analyte detecting member is aligned with the instrument front-end aperture. A spherical bearing surface on the instrument would depress upon the disposable cartridge in the region of the planar valve of the respective penetrating member/analyte detecting member. The depression of the bearing surface on the planar valve causes a flexing of the valve to its open position or "unpinching position". This provides a free channel flow between the mesh front end capture structure and the analyte detecting members.

Figures 52 and 53 show detail of one embodiment : a shaped and formed piece of plastic on a planar surface. The molding of the plastic forms a valvular structure 600 whereas it can be allowed to remain normally on or normally off. The plastic is shaped as to take an advantage of the plastics natural relaxation properties. When nothing is placed against the planar plastic surface, the molded structure pinches close as to seal the channel shown. Upon the application of a force, such as that shown, the channel opens up to allow a clear passage between the opening area and the glucose analyte detecting member.

The disclosure relates to concept that utilizes the natural elasticity of the plastic channel and transport structures currently being used. The concept allows a hybrid channel structure comprising of mesh transport and open channel capillary structures. The open area capillary structure is fabricated with a normally closed valve structure. This valve is designed and fabricated in the plastic channel structure of the channel upper or cap layer. The plastic channel valve is opened when the respective penetrating member/analyte detecting member is aligned with the instrument front-end aperture. A spherical bearing surface on the instrument would depress upon the disposable cartridge in the region of the planar valve of the respective penetrating member/analyte detecting member. The depression of the bearing surface on the planar valve causes a flexing of the valve to its open position or "unpinching position". This provides a free channel flow between the mesh front end capture structure and the analyte detecting members.

Referring now to Figures 54 through 59, the technical field of this embodiment relates to the sciences of plastics and its mechanical properties along with the electromechanical mechanisms of solenoids and valves. It relates to the sealing and packaging of diabetic glucose analyte detecting members and its packaging to prevent either physical degradation or electrochemical deviations from its original point of manufacturing calibration or point of reference measurement. The use of plastic material for sealing by flexing is a function of the materials properties of the material itself.

This embodiment is a simplistic method of sealing the glucose test cartridge from the natural degrading properties of atmosphere. The intention of this design is to utilize as much of the existing cartridge structure and the use of the non memory deformation properties of plastic. In one embodiment, the primary structure of this device is a ring, which is installed in the glucose measurement instrumentation. The presence of the ring along with the disposable cartridge creates a tight seal on the cartridge's fluid transport structures between the many individual front end sample capture structures and their respective glucose analyte detecting members. The ring is allowed to open during the individual moments of fluid sample, capture, and transport of the fluid to the respective glucose analyte detecting member.

Referring now to Figure 54, this disclosure is a simplistic method of sealing the glucose test cartridge from the natural degrading properties of atmosphere. The intention of this design is to utilize as much of the existing cartridge structure and the use of the non memory deformation properties of plastic. In one embodiment, the primary structure of this device is a ring 600, which is installed in the glucose measurement instrumentation. The presence of the ring along with the disposable cartridge creates a tight seal on the cartridge's fluid transport structures between the many individual front end sample capture structures and their respective glucose analyte detecting members. The ring is allowed to open during the individual moments of fluid sample, capture, and transport of the fluid to the respective glucose analyte detecting member.

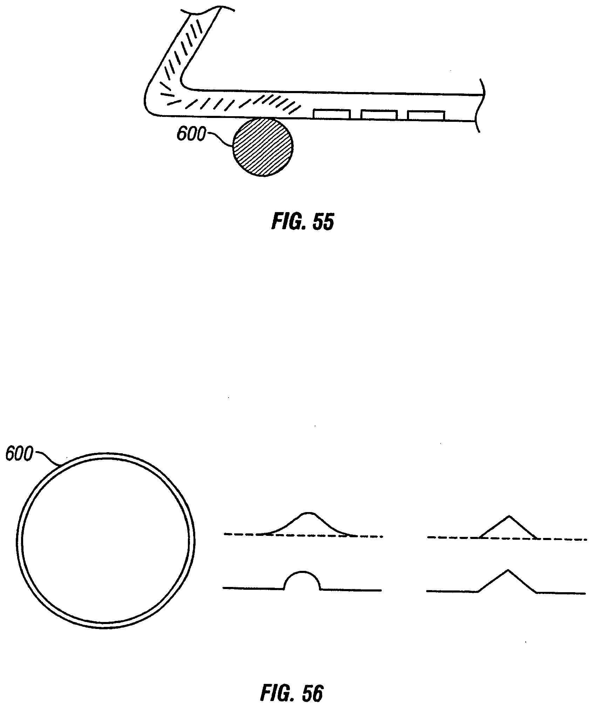

Figures 54 and 55 pertain to the side view or cross section of the ring 60 against the plastic or other substrace surface, either in its normally open position and next in its forced close position as shown with the ring 600 forced against it.

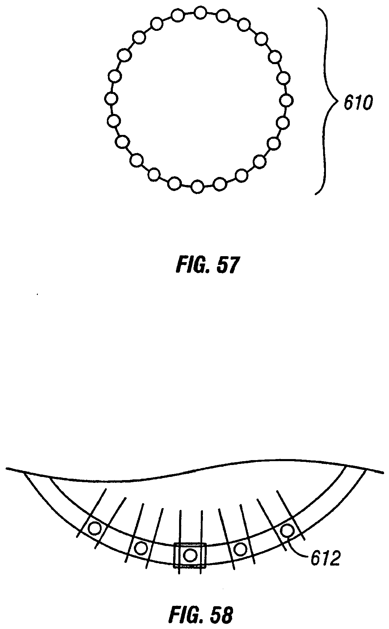

Figure 56 shows another embodiment with either the use of a continuous ring structure 600 with either a round or knife edge to seal the channel in the closed position. Figure 57 shows the use of a "ball bearing" ring 610 is shown. Each ball bearing 612 (as seen in Figure 58) is affixed at a known position as to depress directly onto each individual transport structure channel in a designated location. In this embodiment only in one position is the ball bearing replaced with a solenoid actuated valve for opening the closed channel at time of sample and capture. All the tubes may be sealed by the bearings. There is not enough of a time for contamination. Bearing occludes all but one channel. When the user is done, nothing is rotated until the next is ready for lancing. A ring may be used too (a ring with a slot). This is nondisposable and built onto housing. The package protects the sensing device prior to insertion into the device.

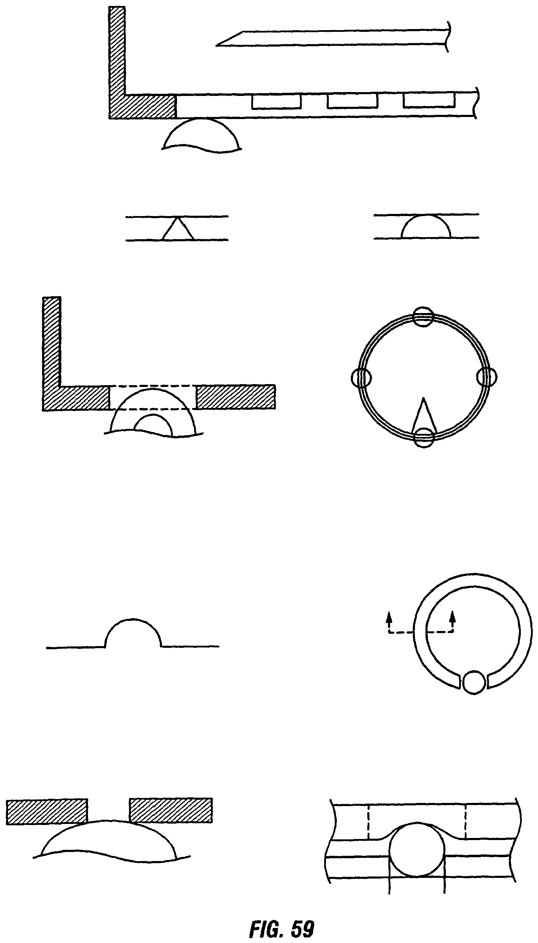

Figure 59 shows other methods and embodiments

In some embodiments the objective of this disclosure is to utilize as few moving parts as possible as to obtain a high reliability for the cartridge. The primary problem is the gradual degradation of the glucose analyte detecting member as the glucose oxidase enzyme is exposed to the ambient atmosphere. When the cartridge is finally measured in the manufacturing process as to establish the measurement calibration or reference point, the cartridge is sealed in a air tight container to deter any deviations in the analyte detecting member chemistry. When the cartridge is ready to be installed in the instrument, the cartridge is removed from the bag or other storage container at which it is then exposed to the atmosphere. However, the cartridge is placed in the instrument with all the sampling transport channels normally open. Again, allowing the degrading properties of the atmosphere to cause the glucose analyte detecting member to degrade. But by design, there will be a capillary channel area that will be wide and long enough to allow a rolling or sliding surface to temporarily deform the plastic cover layer as to produce an air tight seal between the sample capture structure and the glucose analyte detecting member.

This sealing can be by a ring like structure 600 as described above or a knife like structure also described above. In some embodiments, the sealing structure 600 may also be coated with a antifriction film as to allow the cartridge to smooth and uniformly slide along the continuous ring like sealing structure or even the ball bearing surface described earlier. Upon time of operation, the ring 600 at a selected sector can be lifted temporarily as to allow fluid transport to exist between the front-end sample capture structure and the glucose analyte detecting member. In one embodiment, the worst case exposure of the individual glucose analyte detecting members can range from no exposure to a maximum of (say 5 minutes per sample capture procedure) 51 times 5 minutes, resulting in a total time difference of zero time in the beginning of a cartridge to 2 and a quarter hours for the last cartridge. This range of degradation times should be adequate to allow stabilize results in the glucose measurements. In addition, in order to minimize each individual analyte detecting member exposure, a small drying agent can be also placed in each individual cartridge channel, out of the way of the blood flow.

In one embodiment, the intention of this design is to utilize as much of the existing cartridge structure and the use of the non memory deformation properties of plastic. The primary structure of this device is a ring, which is installed in the glucose measurement instrumentation. The presence of the ring along with the disposable cartridge creates a tight seal on the cartridge's fluid transport structures between the many individual front end sample capture structures and their respective glucose analyte detecting members. The ring is allowed to open during the individual moments of fluid sample, capture, and transport of the fluid to the respective glucose analyte detecting member.

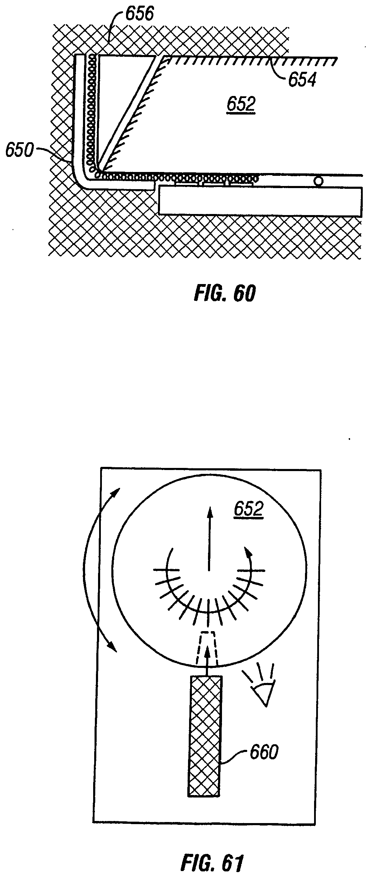

Referring now to Figures 60 to 61, embodiments relate to the ability to allow sterility and air tightness maintenance and servo motor application to allow proper placement of the respective penetrating member for punch and launch. The problem is the maintenance of total penetrating member sterility and the maintenance of sir tightness of the glucose analyte detecting member which can be solved by the application of an independent opening "punch" mechanism and servo control of the respective penetrating member and glucose analyte detecting member which allows placement of the respective penetrating member in the punch position and then subsequently to the launch and use position. The present disclosure may involve the use of heat staked foil/coating to allow the maintenance of sterility of the individual penetrating member unit. This may also include servomechanisms for the exact placement of structures by digital encoding.

Referring now to the embodiment of Figure 60, the sensing device 650 is built around a penetrating member disc 652. In the present embodiment, there is a foil 654 over a foil 656. This provides separate sealing for the analyte sensing devices and the penetrating members in disc 652.

Referring now to Figure 61, the cartridge or disc 652 may be rotated to bring an active cavity in the cartridge to a neutral position and use a vertical or lateral punch 660 to remove the foil. It takes the mechanism for opening and puts it to an available area in the. Servo is for how to open one or both foils. The penetrating member in cartridge 652 is not used to open the foil. Instead, punch 660 is used. After punching of the foil, the disc 652 may be rotated to bring the active cavity with the active penetrating member back into position for launch. In one embodiment, the disc 652 may be encoded such as but not limited to an (LED and detector) to rotate the disc back to the desired position. A servo, other electric actuation, pneumatic actuation, or computer controllable actuation device may be used to rotate the disc 652. Alternatively, the user positions the active chamber in a desired position and the servo always move the chamber or cavity in the active position to the punch position and back again (thus not using the encoding).

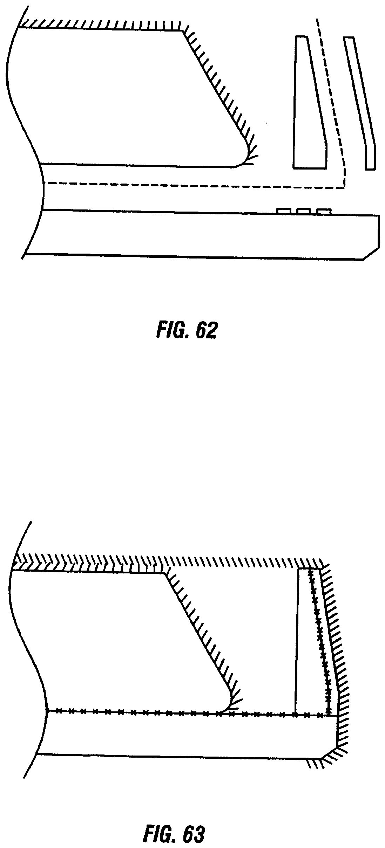

Referring now to Figures 62 and 63, improved embodiments of the devices of Figures 60 and 61 are shown. The sterility of the penetrating member is desired throughout the fabrication and user application. In addition, in one embodiment, the glucose analyte detecting member is desired to be sealed in an air or non-oxidizing environment through out the user application. This is also encumbered by the desire for the sealing material to be removed from the blood sampling and penetrating member pathway during the duration of the individual penetrating member and glucose analyte detecting member use application. This disclosure uses many different disclosed and developed methodologies, and established material converting techniques to solve this diverse problem.

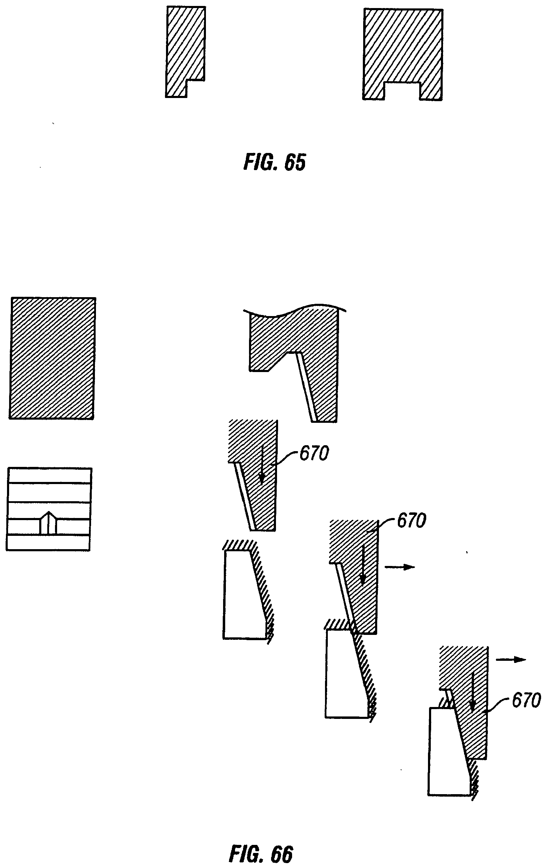

Building on the concepts shown in Figures 60 to 61, a similar device is shown in Figures 65 to 66, except that the punch will come in a slanted angle. The problem is the ability to open a sterile and air tight glucose analyte detecting member and penetrating member structure without possible obstruction of the sample capture front end structure of the analyte detecting member and penetrating member structure and is solved by vertical opening using a multivertical punch mechanism. In one embodiment, the punch travels along an angled path. In another embodiment, the punch 660 may have an inner prong that will dig into the foil. This is a punch solution to the foil solution. Another option is to have two punches near the front.

In one embodiment, some of the design objectives for this concept are: 1). Achieving a hermetic seal to prevent glucose analyte detecting member degradation. 2). Maintaining penetrating member sterility and contamination isolation. 3). Leveraging from existing L1 design. 4). Providing unobstructed sample / capture area.

In some embodiments, the device may include: 1). Lollipop micro fluidic sample / capture structure works. Fluid sample volume, depending upon the PSA heights, range from 200 nL to 400 nL. 2). Penetrating member cartridge is sterile and sealed. 3). Penetrating member foil punch works. 4). M2 Inventus test strip works. 5). Foil methodology established. 6). Front End sample / capture with wicking and sipping structures demonstrated with lancing integrated on the laboratory test stand. The present disclosure relates to: 1). Sealing the front-end sample / capture to provide glucose stability. 2). Maintaining an unobstructed front end after the seal is removed.

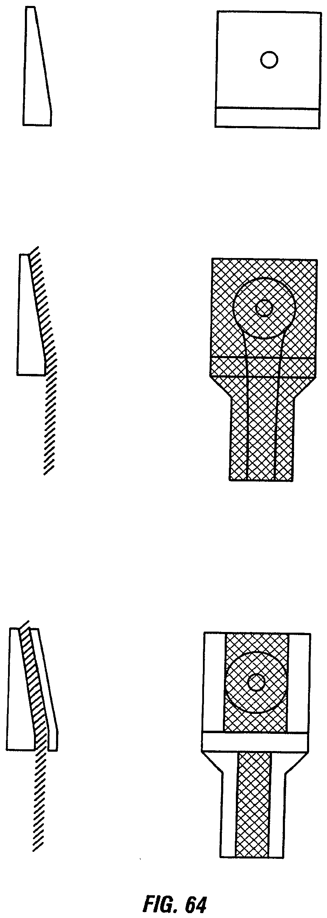

In one embodiment, as described in more detail in Figures 60 and 61,a concept was developed as a carrier within a carrier, which used a horizontal punch. This horizontal punch mechanism created a set of sample / capture area obstruction problems and possibly the need for a servomotor. Additional details are shown in Figures 62 to 64.

Referring now to Figures 65 and 66, the present embodiment shown in the figures presents a vertical punch solution to open the sample / capture cartridge utilizing the cartridge within a cartridge concept. It is a leveraged concept off the current developed solutions, which preserves the integrity of the penetrating member sterility and stability of the glucose analyte detecting member. It also allows the use of the developed "Lollipop" micro fluidic structure for sample, capture, and transport of the nano liter volumes of blood to the glucose analyte detecting members.

The fabrication of this structure can be seen in the attached manufacturing schematic. In addition, the method of the punching protocol and operation is diagrammatically shown in the above drawings.

Figure 66 shows a punch 670 traveling along an angled path to remove foil or sterility barrier 672 from the front surface.

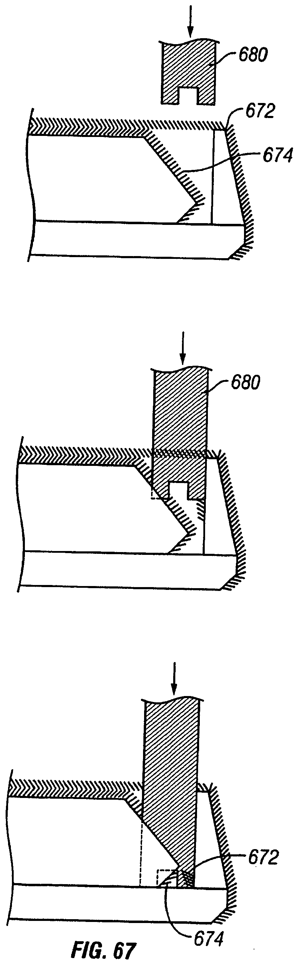

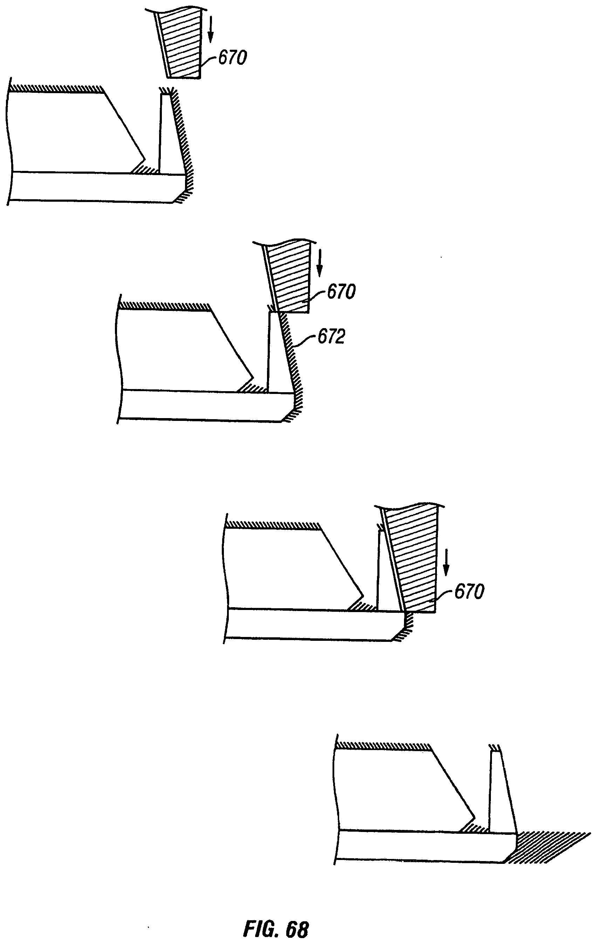

Figure 67 shows how a two punch embodiment works. The punch 680 punches through a first foil 672 and then a second foil 674. Figure 68 is similar to Figure 66 in that a punch 670 removes a portion of the foil 672 that remains on the front surface and may be covering the penetrating member exit.

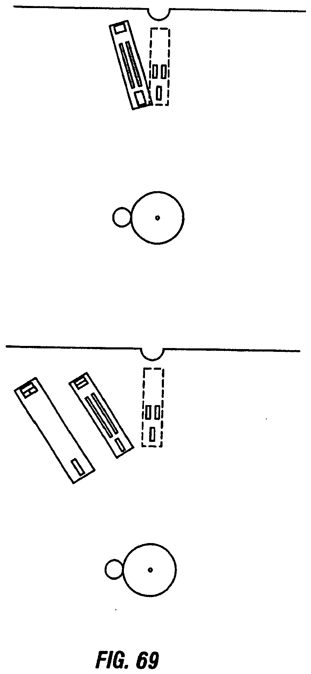

Figure 69 shows top down view of various punches and their positions. There may be a single punch or a double punch as shown in the various embodiments shown in the figures.

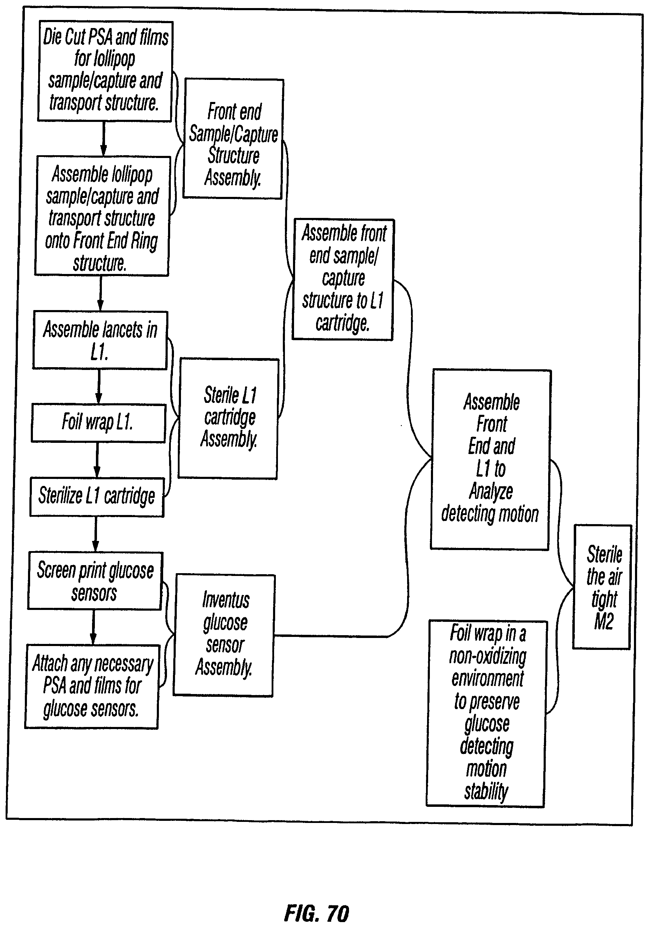

Figure 70 shows one embodiment of the manufacturing steps for use with the embodiments disclosed herein.

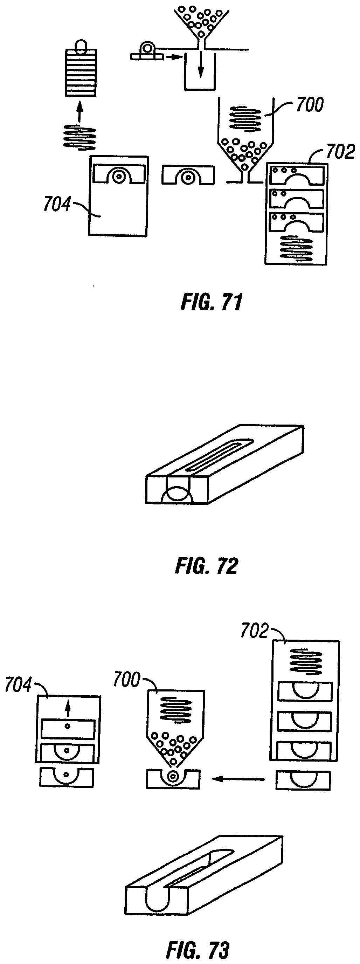

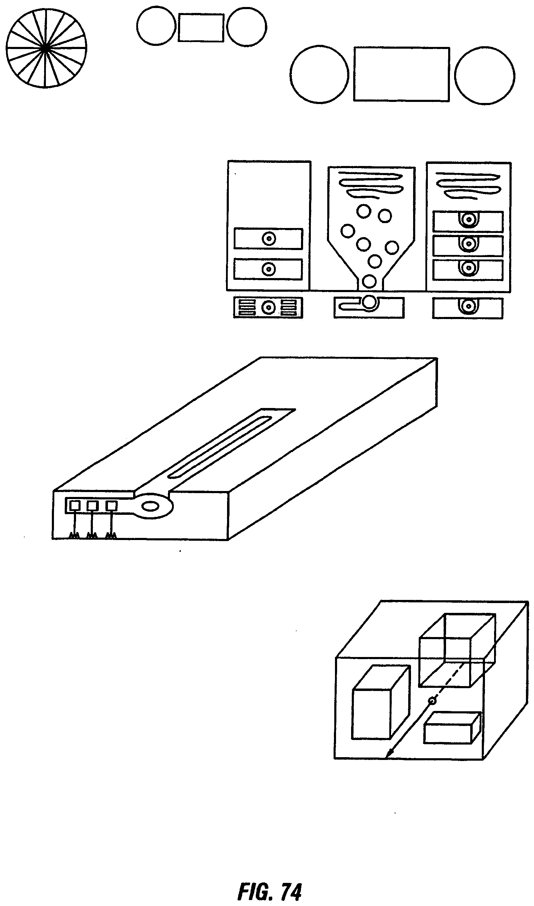

Referring now to Figures 71 to 75, the technical field of this embodiment relates to lancing, sampling, sensing, and disposable modular structures as to allow the maintenance of air tightness and sterility. If a cartridge did not exist for housing a multiple penetrating member/multiple analyte measurement device, how would one set up the instrument. Big cost was the packaging. This is a bulk approach and could be an institutional device. For some embodiments, the problem is the maintenance of modularity, sterility, and air tightness and is solved by using the plastic modular molding and binning of both existing glucose test strips and penetrating members.

Referring now to Figure 71, for some embodiments the concept utilizes three distinct chambers: New penetrating members 700, new glucose cartridges 702, and used penetrating member and glucose cartridges 704. Each chamber is sealed from the others through a series of sliding doors such that integrity of the penetrating members sterility and the glucose analyte detecting members stability is insured.

As seen in Figure 72, the concept is intended to allow the manufacturer of the glucose cartridges and penetrating members to produce them as is and the packaging to be as simple as possible.

Referring now to Figure 73, it should be noted that the dispensing of the penetrating members leverages upon the dispensed new glucose analyte detecting member. The penetrating member may fall automatically in place on the glucose analyte detecting member and is readied for firing. Upon completion of the penetrating member, sample and capture, both penetrating member and glucose analyte detecting member are removed and stored away in a "used" cartridge for isolation. It should be understood that some embodiments may have more bins or less bins. Some may only have a penetrating member bin and a cartridge bin. Others may have a penetrating member bin, a cartridge bin, a test strip bin and a used bin. Any single or multiple combination of the elements above may be used.

Embodiments may include the following features or advantages: thin device 3mm, width can be thin by wrapping mesh over analyte measurement device. Length is controlled by length of lancet. Can analyse cartridges after use. Old is both lancets and analyte measurement devices together. Sterilization can happen separately Transfer mechanism, which assembles lancet to cassette and delivers it to the driver. This module can be the guide bearing Good way to feed it into the gripper. Could be bearing system, get blood and then throw it away. Separation of used from unused. Separate lancet and strips. Cartridge to solve sterility and air seal. Gets away from limited space of disk. Collects old contaminated structure.

Referring now to Figures 76 to 80, this embodiment of the present invention solves the packaging constraints for the disposable cartridge 800 by providing a sliding valve, which closes off the blood transfer path and the air vent to the analyte detecting member.

Referring now to Figure 77, described below are all elements that make a sterile barrier around the penetrating member 812, what maintains the moisture barrier and then how it is opened and closed. In one embodiment, the central feature of the concept is the sipper component 810 an injection molded polymer about 2 x 2 5 mm which sits in the front pocket in front of each analyte detecting member. The sipper 810 forms a sliding valve with two ports in the disk. The air vent and the blood transfer port in the firing position the sipper also forms the finger contact face, the penetrating member passes through it while firing.

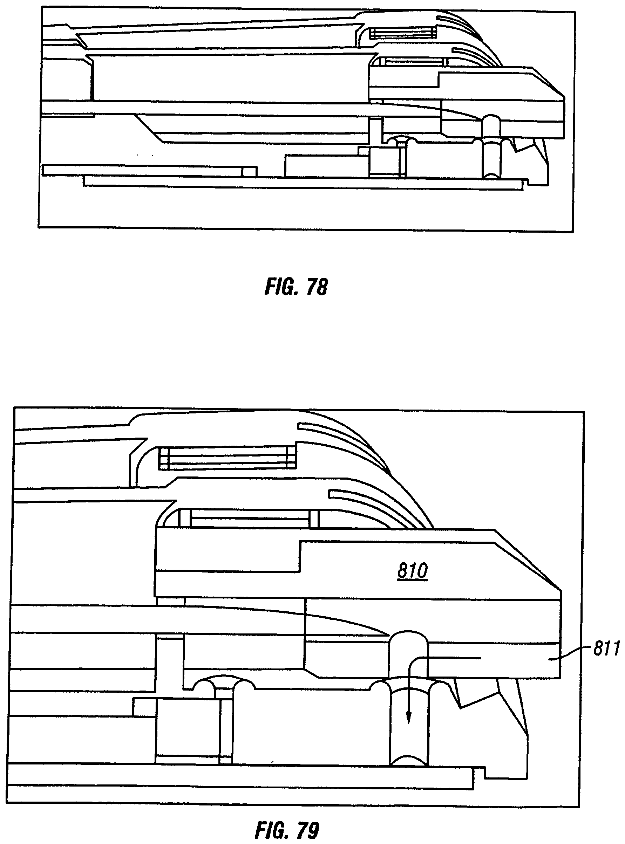

In one embodiment as seen in Figure 77 to 79, the sterile barrier around the penetrating member is comprised by the disk and the aluminum foil lid, and the two ports are closed off by the sipper. That assembly is sterilized as one unit. Subsequently the analyte detecting member substrate is adhesively bonded to the bottom of the disk with desiccant in a cavity in the bottom of the disk. The desiccant is in air communication with the analyte detecting member. To operate the device, first the foil is punched from the main cavity and punched in front of the sipper. The front punch could be a combination of L1 front punch and the plough concept. It could be punched into the storage cavity. As seen in Figure 78, a mechanism in the instrument pushes the sipper until it comes to a stop defined by a feature on the bottom of the sipper 810 and in the disk pocket at the front.

Referring now to Figure 79, the finger is placed on the front 814 of the sipper 810 the penetrating member is fired and blood flows along the capillary channel (penetrating member channel) and down the sipper transfer port and disk transfer port tot the analyte detecting member channel formed by the analyte detecting member substrate the bottom of the disk and the PSA boundaries. Air vents through the air vent into the front cavity and out past the punched foil. There will be some mechanism to retrench the sipper valve into the front pocket.

The sipper 810 may include a feature to prevent it from lifting up. The disk has a retaining feature preventing the sipper lifting up and this feature could be created by cold forming the disk material. Blood Volume: Volume through Sipper to transfer port, down transfer port, through hole in Disk, along analyte detecting member channel until 3rd electrode is covered is about 0.74 microliter. It should be understood that the chamber sizes and volumes such as 1 microliter, 0.5 microliter, 0.4 microliter, 0.3 microliter, 0.2 microliter, and the like may be used.

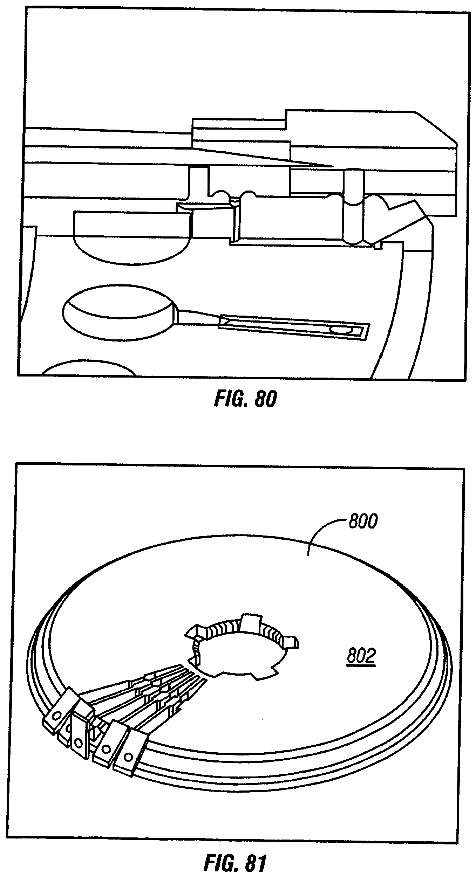

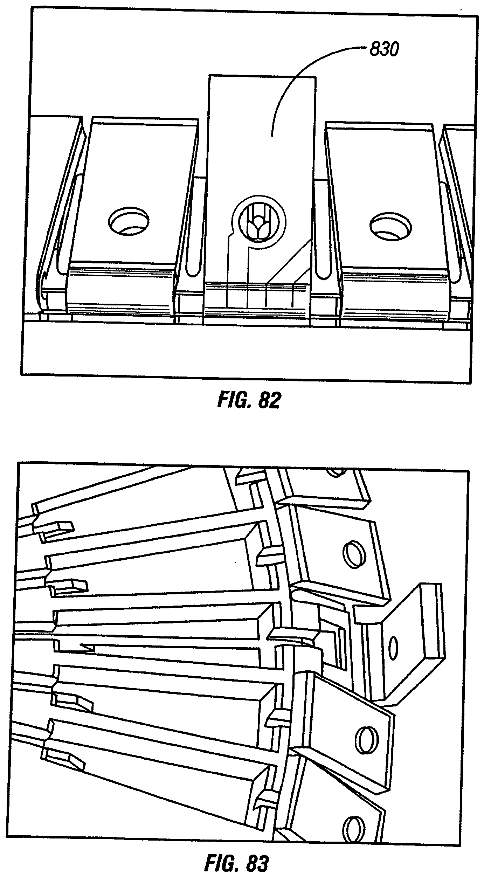

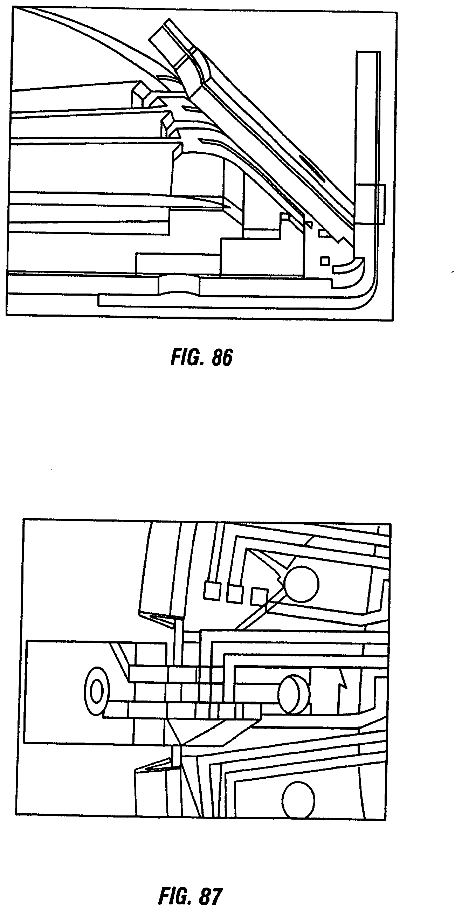

Referring now to Figures 81 to 88, a still further embodiment will now be described. In one embodiment, the enabling feature of the hinged valve concept is a flat kinking tube caused by the differential movement of two membranes. This concept comprises the sterile penetrating member disk 800 with the analyte detecting member disk added as a separate assembly.

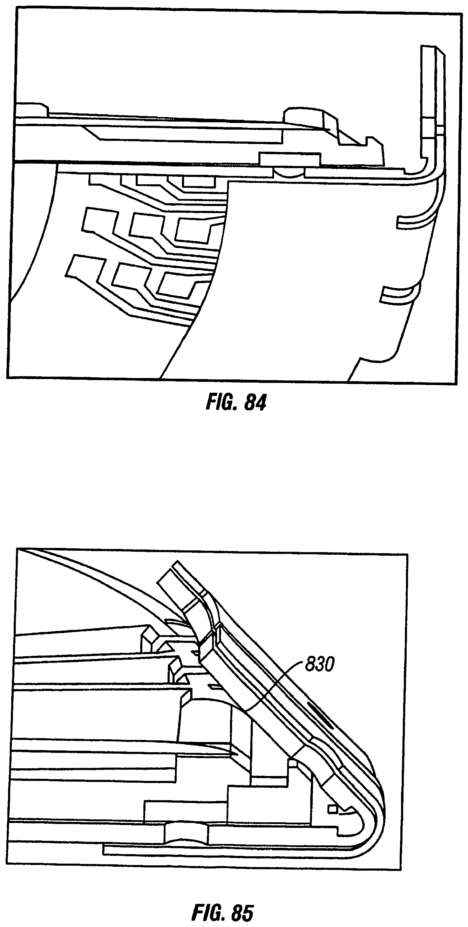

As seen in Figure 81, the disk 800 and the foil 802 whose design remains unchanged and creates the sterile barrier. In one embodiment (as seen in cross-section of Figure 84) the analyte detecting member substrate and the cover film laminated with a PSA layer, which forms the channel sidewalls, create the analyte detecting member chamber seal and the air vent hole in the analyte detecting member substrate communicates with a blind hole in the disk component, this cavity may be filled with desiccant. The closed hinged valve 830 seals the front end of the analyte detecting member channel with the hinge valve tab in its closed position. To operate a mechanism in the instrument (cf "Boot mechanism") moves the tab forwards to its open position and this movement opens the hinge valve channel by unkinking it. The foil is punched in the main and front pockets the penetrating member is fired through the open hole in the front tab into the finger and retracts. The penetrating member does not touch any part of the analyte detecting member and so maintains its sterility. Blood flows into the hole in the cover layer of the tab and into the annular groove around this hole. From the annular groove it flows down the channel past the hinge valve into the analyte detecting member channel. Air is vented by returning along the air vent channel (parallel to the analyte detecting member channel), through the hinged valve to the atmosphere.

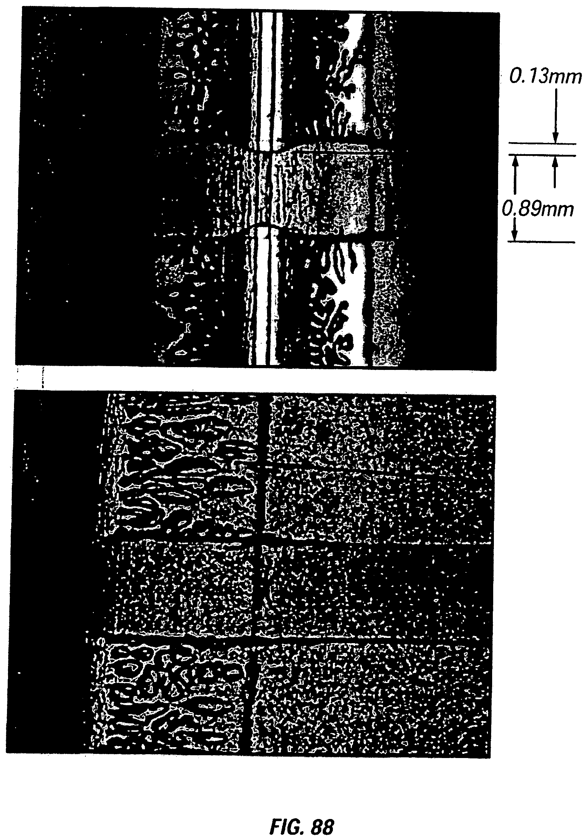

### Figure 88 shows opening and closing depending on the bend angle

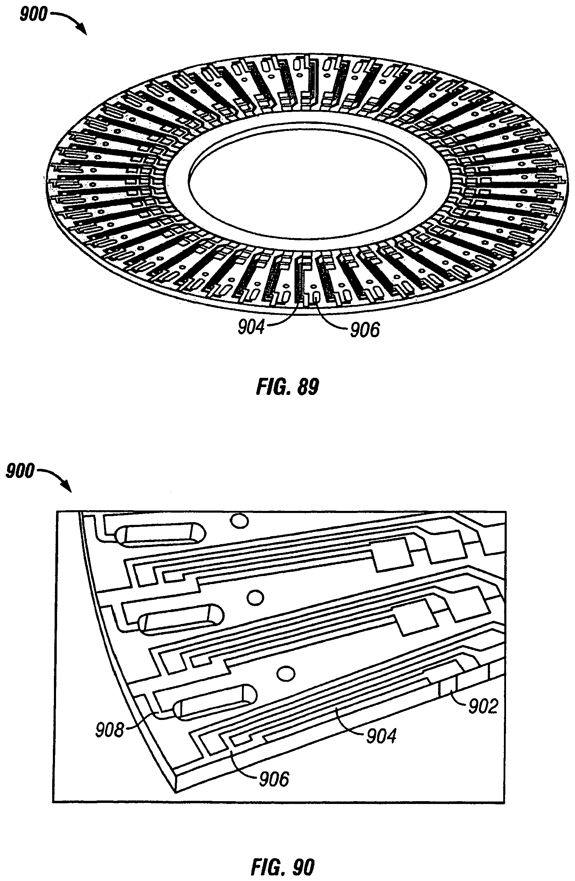

Referring now to Figure 89, a still further embodiment of the present invention will now be described. Figure 89 shows a substrate 900 with a plurality of contact pads 902, electrical leads 904 and analyte measurement members 906. The problem is capturing spontaneous blood flow from a lancing event and measuring the glucose level in this blood. In some embodiment, The problem is solved by:
1. Using a wicking mesh structure to capture the blood and transport it to sensing electrodes
2. Aligning the mesh with the lancing device such that the mesh is positioned to quickly capture blood from the lancing site
3. Including a reservoir structure to capture excess blood
4. Designing channel structures such that blood will first flow over the electrodes, and then remain motionless over the electrodes while excess blood then flows into the reservoir
5. Including desiccant to keep the glucose analyte detecting members dry before use
6. Including a vent chamber where displaced air can flow into without escaping to the environment
7. Sealing the entire device in a foil to keep the analyte detecting members hermetically sealed before use
8. Using a punch to open up the foil before use.

Embodiments may be based on a two-disk concept with a front seal and a system for punching, separating lancet from analyte detecting member, with or without mesh and PSA for sticking it all together.

Glucose sensing electrodes are printed onto a suitable substrate. This substrate has a hydrophobic surface, except for a hydrophilic surface treatment applied to define fluid channels. The substrate has holes punched through it to create a fluid reservoir and a vent hole. In this embodiment, the substrate is a circular disc with 51 sets of analyte detecting members, reservoirs, and vents. Another possible embodiment is the use of a ribbon-like substrate which can be wrapped in a circle around a disc.

Referring now to : The analyte detecting member disc with electrodes (black), hydrophilic coating (purple), and fluid reservoirs (the oval-shaped holes).

Referring now to Figure 90, in one embodiment, A section of one embodiment of the substrate 900 is shown. On this substrate fluid channels 908 are defined by printing Pressure Sensitive Adhesive (PSA). These fluid channels are on the order of tens of microns deep and hundreds of microns wide.

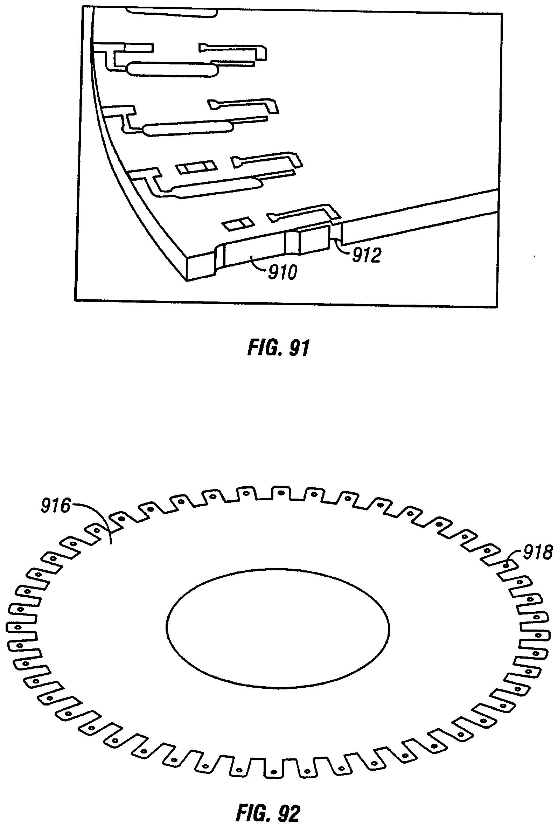

Referring now to Figure 91, in one embodiment, a section view showing the PSA (red) is shown. Here the section view cuts through the fluid reservoir 910 and vent hole 912. In one embodiment, hydrophobic channels in the PSA lead to the vent hole.

A hydrophilic, wicking mesh is cut to fit on top of the substrate. The mesh extends beyond the edge of the substrate and includes holes to allow for passage of a lancet.

Referring now to Figure 92, in one embodiment, a hydrophilic mesh 916 cut to fit on top of the glucose analyte detecting member substrate is shown. The "gear teeth" 918 extend beyond the edge of the substrate, and include one hole per tooth to allow for passage of a lancet.

Fluid channels 920 are cut into two pieces of PSA, which are then impregnated into the mesh from the top and bottom surfaces. The impregnation of the PSA confines fluid flow to the channels.

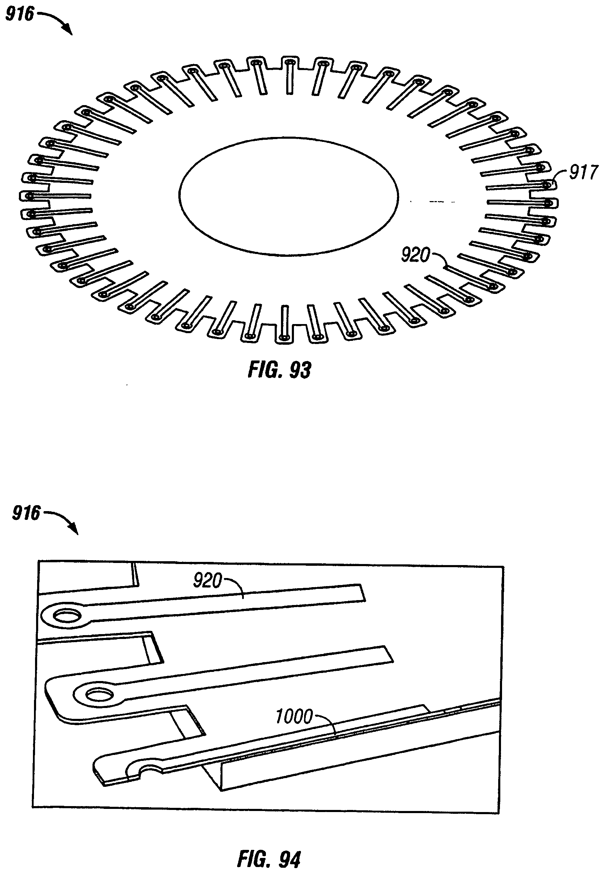

Referring now to Figure 93, in one embodiment, PSA (red) impregnated into the mesh 916 is shown in Figure 93.

This mesh/PSA disc is placed on top of the substrate with the channels in the mesh aligned with the electrodes. The mesh and the substrate are bonded together via the PSA surfaces on the bottom of the mesh and the top of the substrate. In this embodiment, a hole in the substrate is used to capture excess blood. Another possibility is to not use a hole, and to make the channels in the mesh and/or on the substrate large enough such that excess blood can be stored in the channels themselves.

Referring now to Figure 94, in one embodiment, section view of the PSA-impregnated mesh 916 on top of the substrate is shown.

A previously manufactured sealed and sterilized penetrating member disc is then bonded to the top of the mesh (via the PSA in the mesh), with the lancet centerlines directly above the electrodes and their fluid channels. In some embodiment, the wicking portion only extend to an area 1000 short of covering the electrodes. This hybrid design combines a wicking membrane with a capillary device in the latter half of the passage.

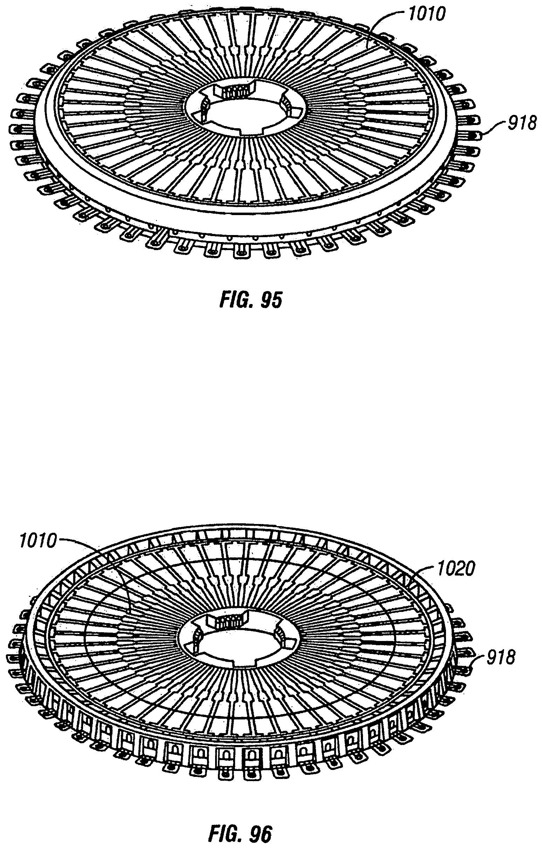

Referring now to Figure 95, in one embodiment, a sealed and sterilized penetrating member disc 1010 placed on top of the mesh 916 and glucose analyte detecting member substrate is shown.

A support ring 1020 is placed around the circumference of the disc. This ring has two purposes, in one embodiment, to support the mesh in a position normal to the path of the lancet and to provide a surface to attach a foil which seals the device off from the environment.

Referring now to Figure 96, in one embodiment, support ring 1020 may be attached around and above the penetrating member disc 1010 is shown.

The mesh "gear teeth" are folded up 90 degrees and attached to the edge of the penetrating member disc and the outer surface of the support ring via the PSA impregnated in the mesh. The mesh is now in a plane normal to the lancet centerline, and the hole in the mesh is aligned with the lancet centerline such that the lancet can proceed through the hole unimpeded.

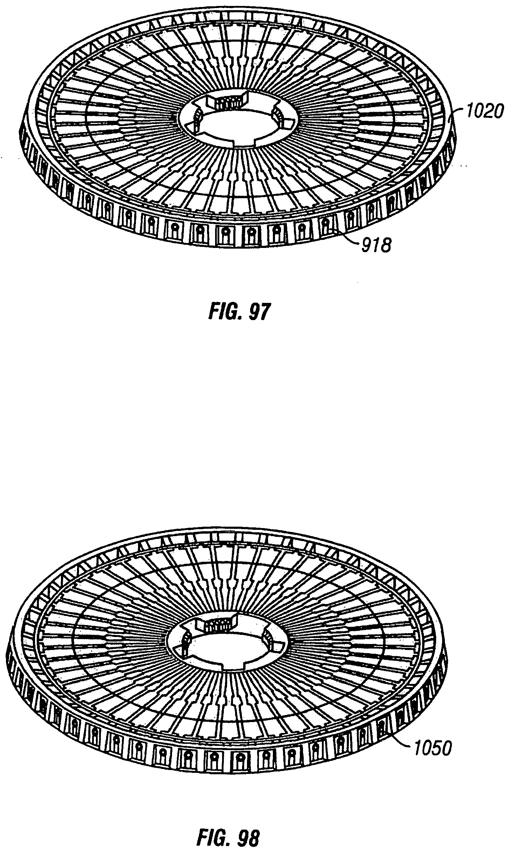

Referring now to Figure 97, in one embodiment, the mesh "gear teeth" 918 folded up is shown. A polymer cover film is applied to the outside surface of the mesh. This polymer film may have a hydrophobic outer surface and a hydrophilic inner surface (the side facing the mesh). The teeth may fold into cutouts in the ring 1020.

Referring now to Figure 98, in one embodiment, mesh 916 covered with a film 1030 is shown.

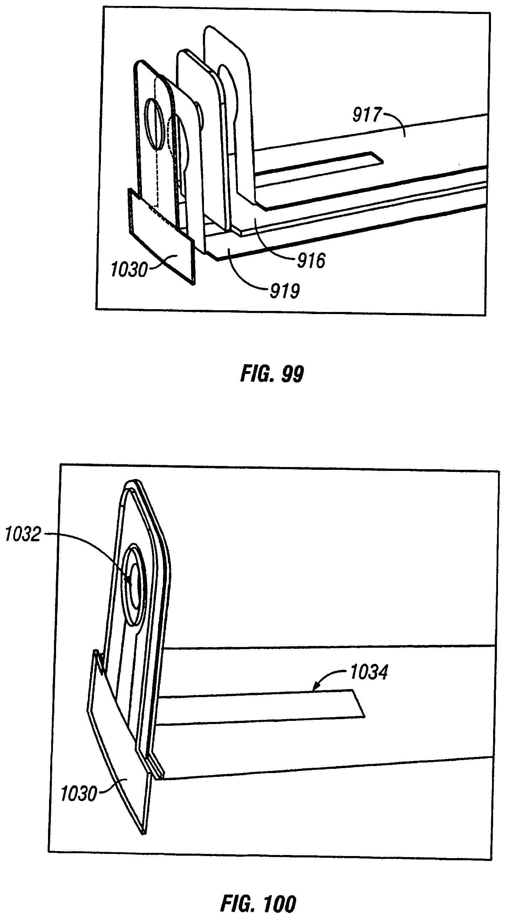

Referring now to Figure 99, in one embodiment, close-up of the film 1030, PSA7, mesh 916, and PSA 919 (exploded) is shown.

Referring now to Figure 100, in one embodiment, an assembled mesh capture structure is shown. The sample may flow from the finger contact area 1032 and enter a chamber 1034.

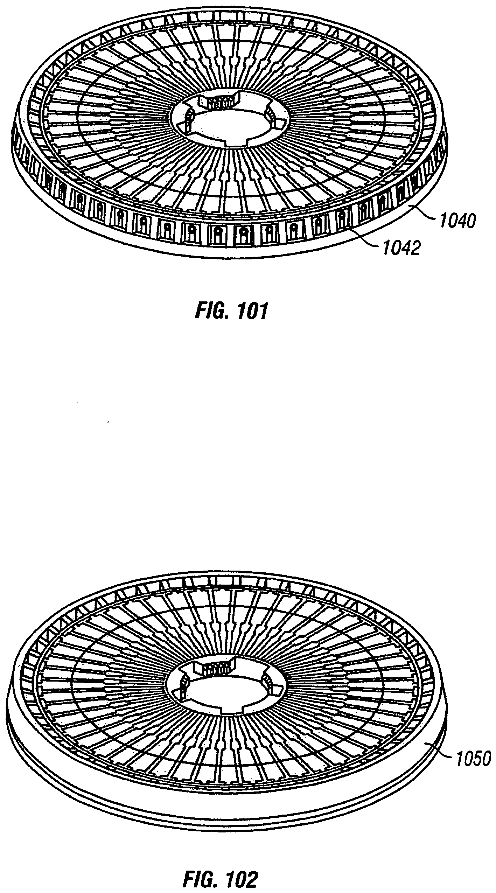

Referring now to Figure 101, in one embodiment, desiccant substrate 1040 attached to bottom of substrate is shown. A plastic part containing individual desiccant chambers 1042 for each set of analyte detecting members is attached to the bottom of the device. These chambers not only contain desiccant, they also provide a venting volume for air in the fluid channels. As blood enters the device, the air which was in the channels will be displaced. If this air is displaced into the environment, then a vent hole is desired, and this vent hole will compromise the sealing of the device during storage unless it is sealed and the seal is broken at the time of use. A simpler solution is to create a sealed volume into which the displaced air can be forced into. If the volume is large enough, the increase in pressure will not be enough to counteract the surface tension forces drawing fluid into the channels. The desiccant chambers include such a volume for the displaced air. In this embodiment, separate desiccant chambers are used for each set of analyte detecting members. Another possible embodiment is to use a single large chamber with desiccant in it that is connected to all of the analyte detecting members.

The outer surfaces of the device are then sealed with foil to protect the analyte detecting members from the environment during storage.

Referring now to Figure 102, in one embodiment, device sealed in foil 1050 is shown. The seal may be a ring or a disc shape.

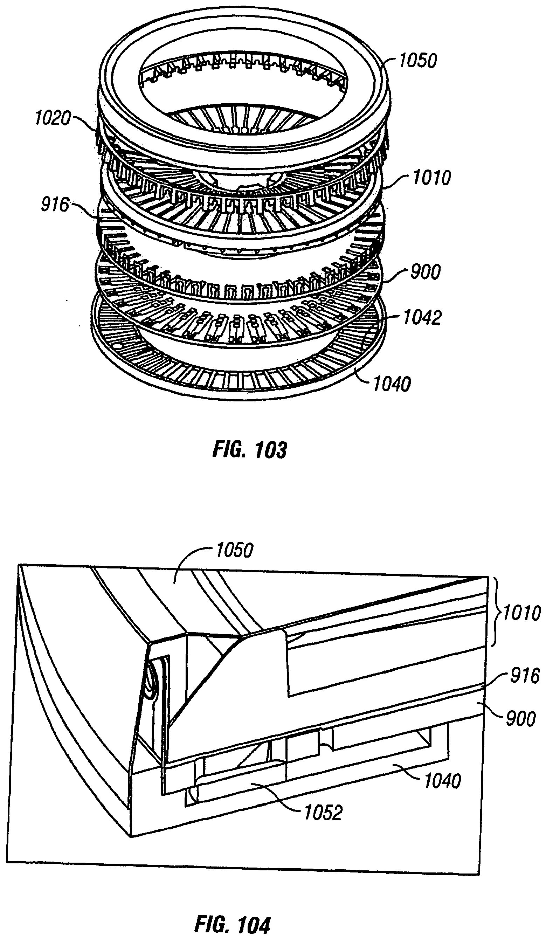

Referring now to Figure 103, in one embodiment, section of the completed device, with the section cut through the lancet centerline is shown.

Referring now to Figure 104, in one embodiment, section cut of the completed device, cut through the fluid reservoir and vent hole is shown. The block 1052 is desiccant.

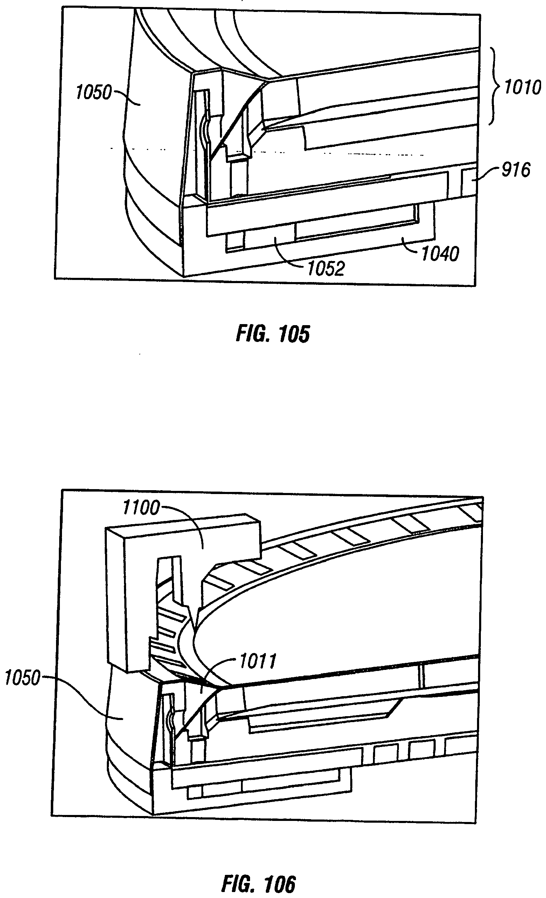

Referring now to Figure 105, in one embodiment, an exploded view of the device is shown with a cross-section taken at a different location.

Before use, the foil sealing one of the glucose sensing structures can be broken (simultaneously with the breaking of the foil sealing the associated lancet). Note that unsealing one pair of a lancet and a glucose sensing structure does not unseal the others on the disc.

Referring now to Figure 106, in one embodiment, punch to open up the foil, shown before use is shown. Figure 106 shows the punch 1100. The punch 1100 is shaped to pierce tow pockets and release the foil 1011 over disc 1010 and foil 1050. The punch 1100 may have two sets of teeth 1102 and 1104.

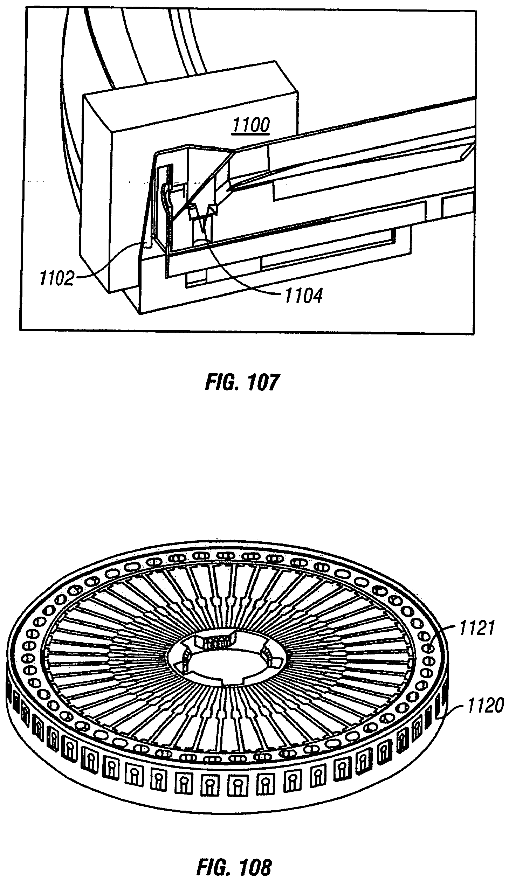

Referring now to Figure 107, in one embodiment, punch shown during use; the foil is visible in the original position.

After the foil 1050 is opened up, the user's finger is placed over the mesh capture structure. The finger is lanced (through the hole in the mesh). Blood from the wound contacts the exposed circle of mesh and is wicked down to the electrodes. The air in the channels is displaced into the desiccant chamber. If excess blood still exists on the outer surface of the mesh after the electrode channel has filled, then this blood will wick into the reservoir built into the substrate and be safely stored.

Referring now to Figures 108 to 134, still further embodiments are described in the figures. In some of these embodiments, they have integrated lancing, blood capture, glucose sensing, and desiccating capability, but the analyte measurement devices are not sealed (in these particular embodiments) from the environment. For example, Figure 108 shows an embodiment where the analyte measurement devices are not sealed from the external environment. The mesh support ring 1120 includes openings 1121 to allow foil on the disc 1010 to be punched.

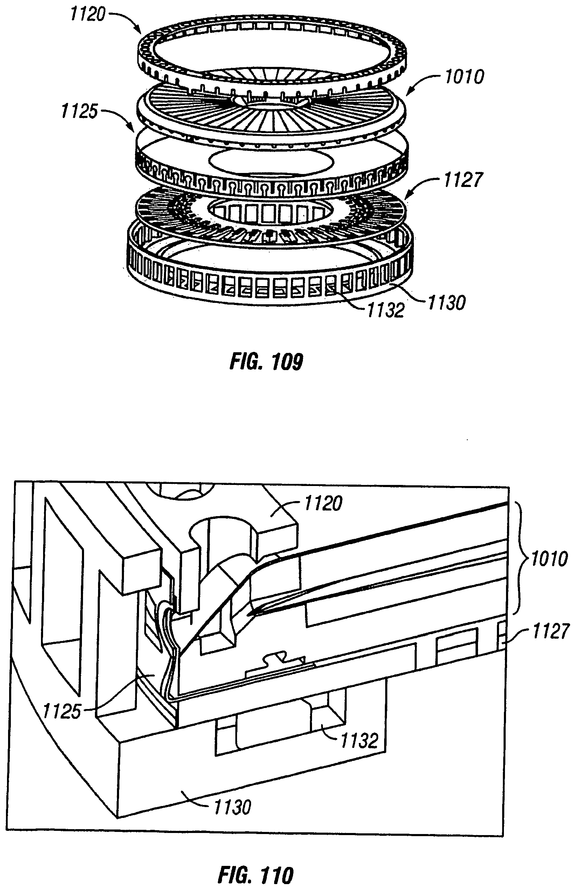

Figure 109 shows an exploded view of the device of Figure 108. Blood capture structures 1125 are shown and lie on top of a substrate 1127 with a plurality of analyte measurement members. A support ring 1130 with desiccant chambers 1132 is coupled to the substrate 1127.

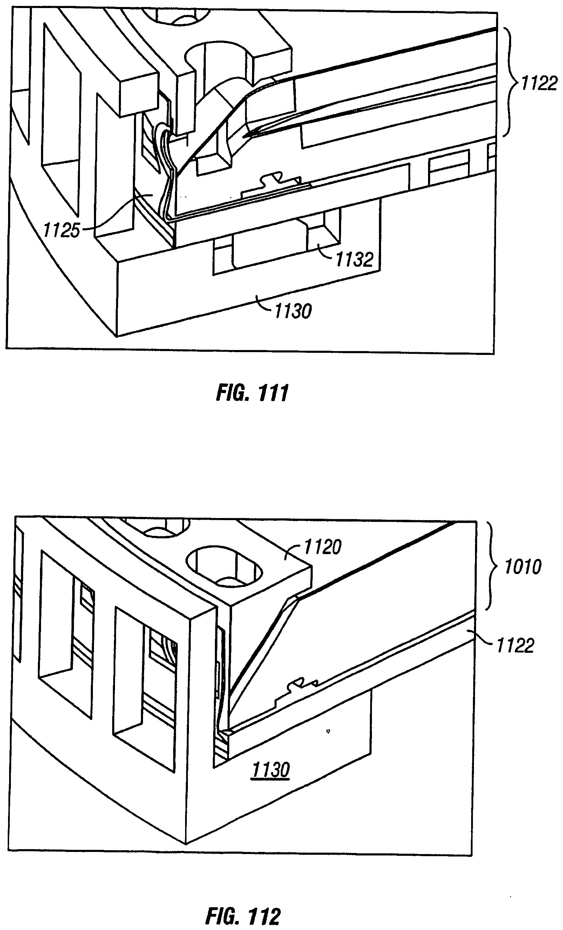

Figure 110 shows a sectional view of the device of Figure 109. Figures 111 and 112 show other sectional views.

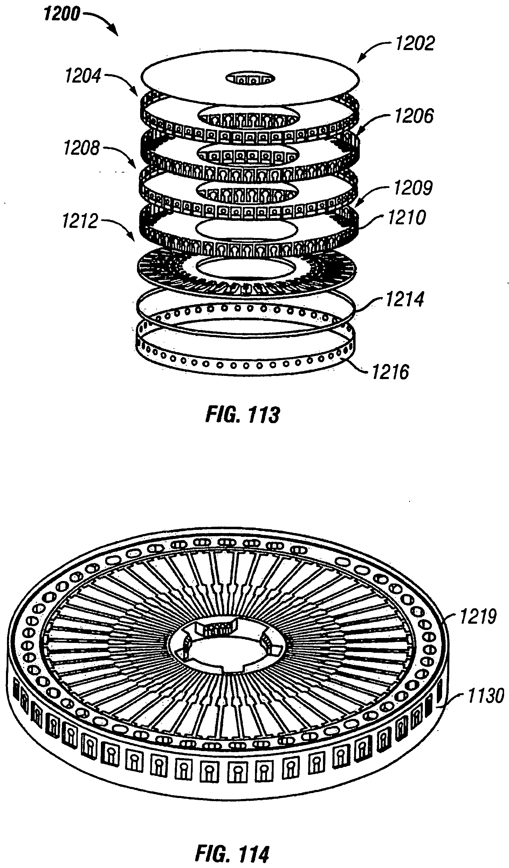

Referring now to Figures 113-117, this embodiment takes the disposable of figures 108-112 and adds an internal, patterned PSA ribbon and two external foils to seal the analyte measurement devices from the environment. The additions include but are not limited to:
a. Instead of one plastic part that provides both a vertical back support for the capture structures and ribs for sealing a foil against, this embodiment has 2 plastic parts. One supports the capture structures, and the other supports the outer foil and holds desiccant.
b. Since we now have 2 plastic parts, these need to be sealed together, so this embodiment incorporates the internal PSA ribbon
c. Instead of 1 sealing foil (in addition to the existing foil on the L1 penetrating member disc), this embodiment uses 2. One foil is a flat, circular disc, and the second is a ribbon wrapped around the circumference.

Figure 113 shows an exploded view of the sample capture structure 1200. A PSA layer 1202 for attaching to a penetrating member disc is provided. A hydrophilic top film 1204 is provided. A PSA layer 1206 is used to defined fluid channels. A hydrophilic mesh 1208 may be provided. A PSA layer is used to define fluid channels. The PSA layer 1209 may have fingers 1210 to follow the shape of the mesh layer. A substrate 1212 is provided with a plurality of analyte measurement members. A PSA ribbon 1214 may be provided on the edge of the substrate 1212. A hydrophilic outer ribbon 1216 may also be provided. Figure 114 shows the PSA layer 1219 between the capture structures and the desiccant ring.

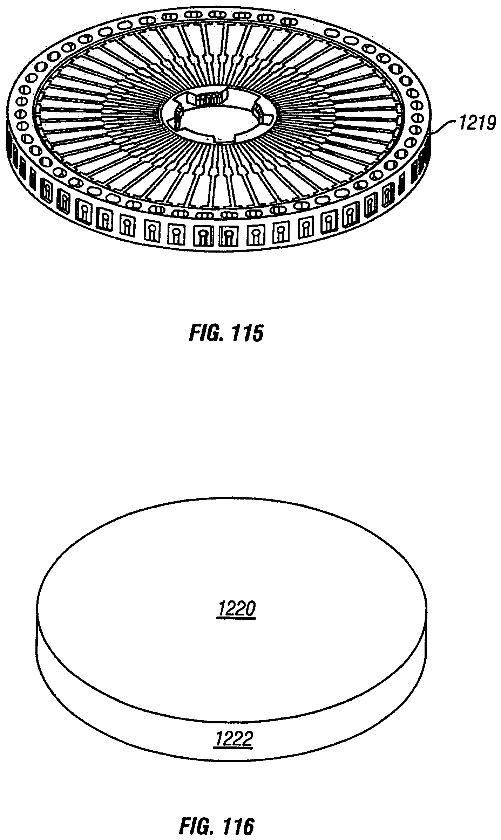

Figure 115 shows an embodiment with the ring 1130 removed to more clearly show layer 1219.

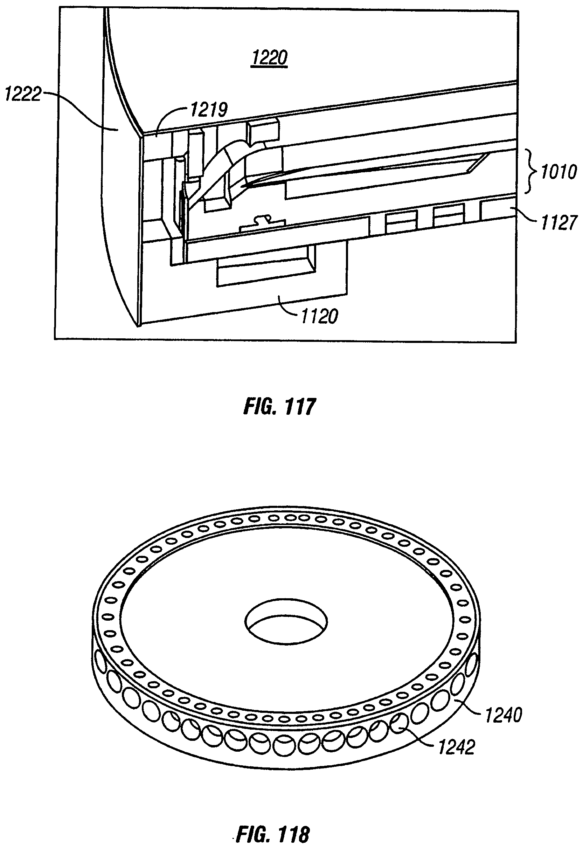

Figure 116 shows and embodiment where two foils 1220 and 1222 are used to seal the device of Figure 114. One may be a top foil in this embodiment in the shape of a disc. The other foil may be a ring or ribbon. Figure 117 shows a cross-section of the embodiment of Figure 116.

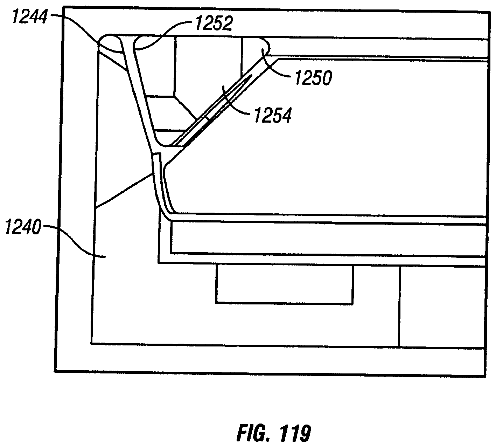

Referring now to the embodiment of Figures 118 and 119, this takes the device of figures 112-117 and changes the mating surfaces of the 3 plastic parts (L1 penetrating member disc, mesh support ring, and desiccant/foil support ring) from cylinders to conical sections. This may allow for easier alignment and better sealing. In the drawings, a 0.25 mm gap may be included between plastic surfaces which will be filled with PSA, just like those in Figures 112-117, except that the PSA is not explicitly shown in the drawings.

Figure 118 shows an outer ring 1240 with openings 1242 concentric with the penetrating members in the disc 1010. Figure 119 shows that desiccant ring 1240 has a angled surface 1244. The mesh support ring 1250 also has non vertical and nonhorizontal surfaces 1252 and 1254 that are angled to facilitate assembly.

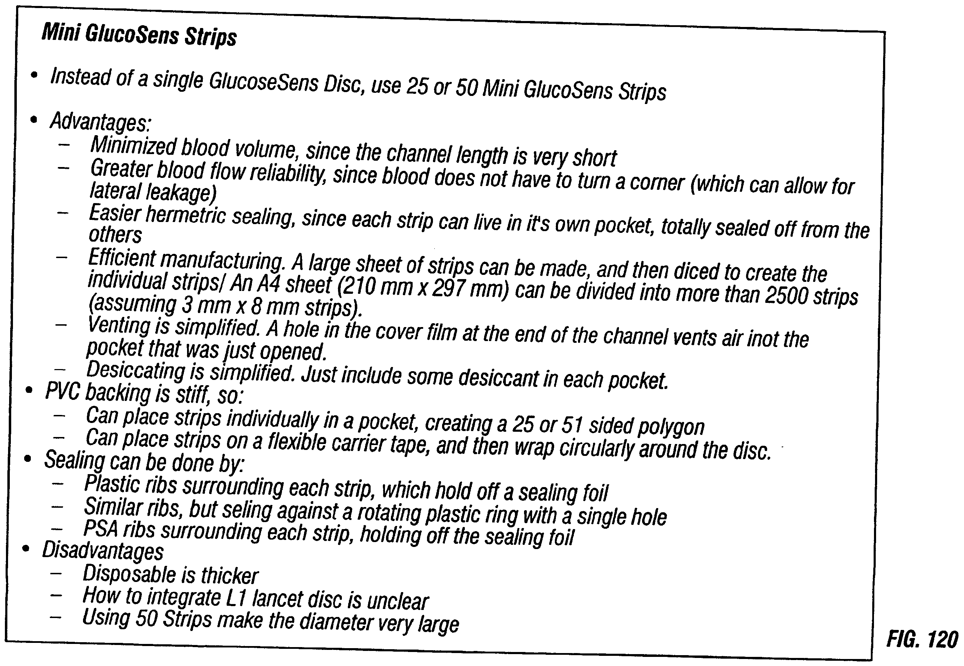

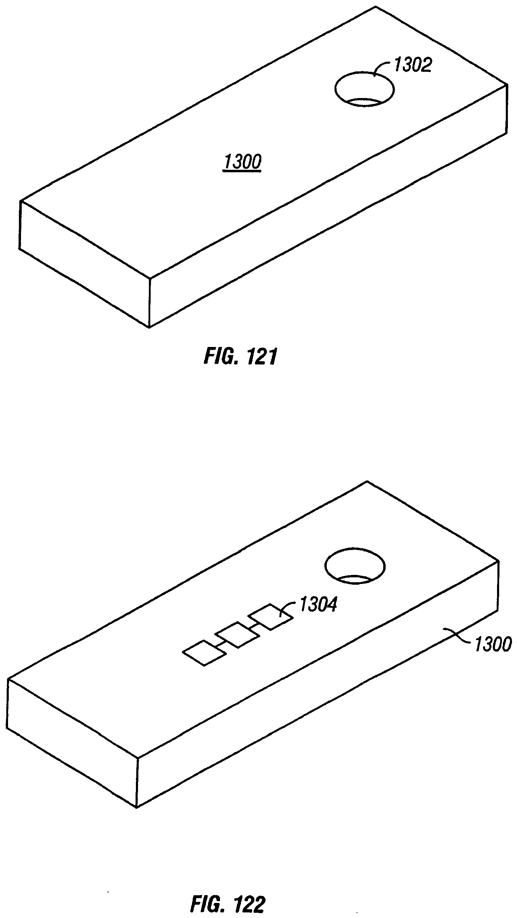

Referring now to Figures 120 and 121, individual test strips may also be formed. Figure 121 shows a substrate 1300 with a opening 1302.

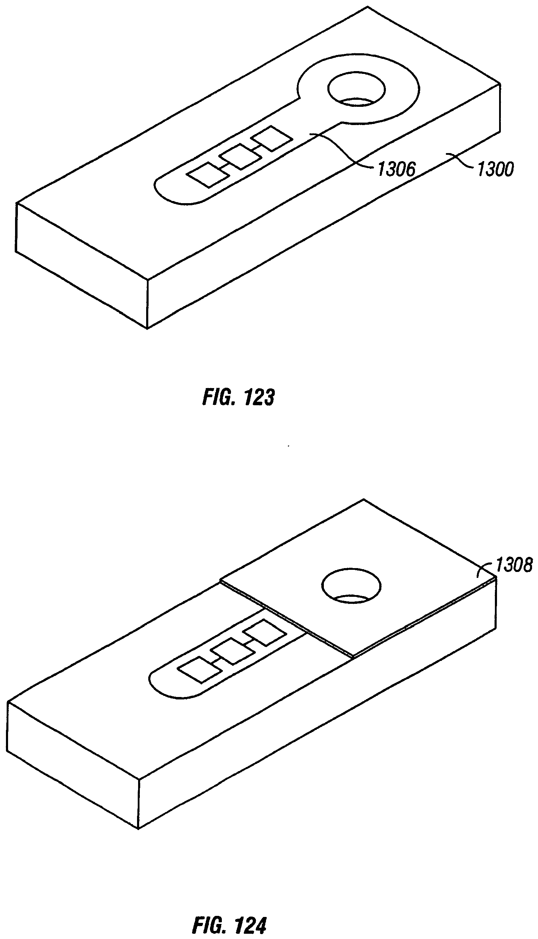

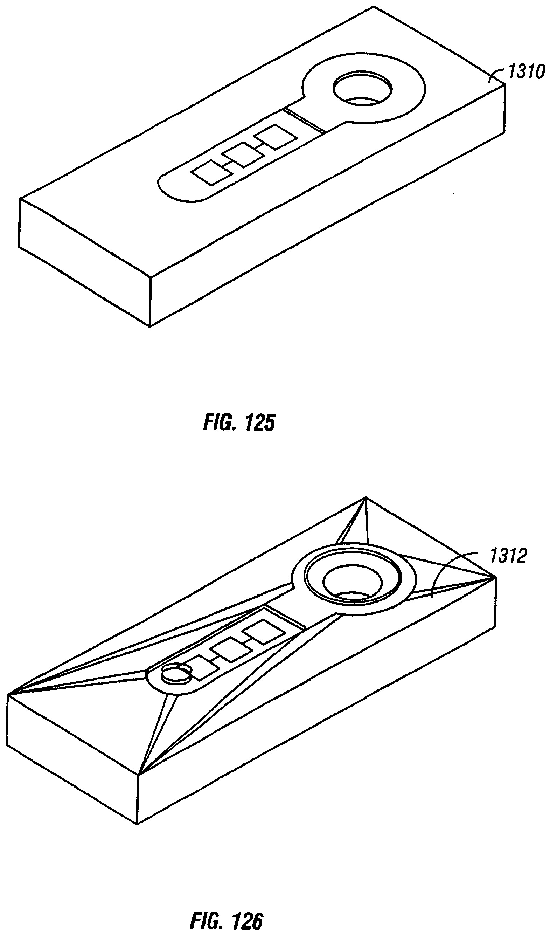

Figure 122 shows three square analyte measurement members 1304 formed on the substrate 1300. Figure 123 shows a hydrophilic coating 1306 applied over the members 1304 and may defined a path from the opening 1302. A hydrophilic mesh 1308 is introduced in Figure 124. A PSA layer 1310 is introduced in Figure 125 to define a fluid channel. A cover film 1312 is applied in Figure 126.

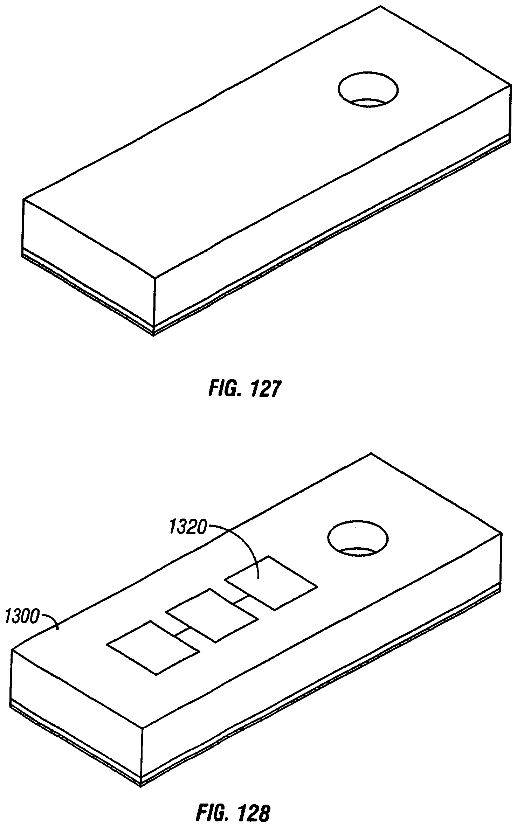

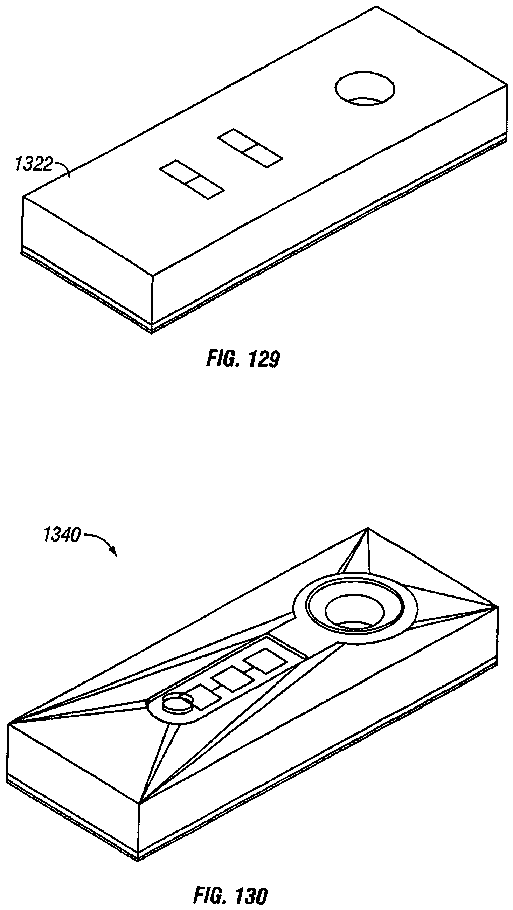

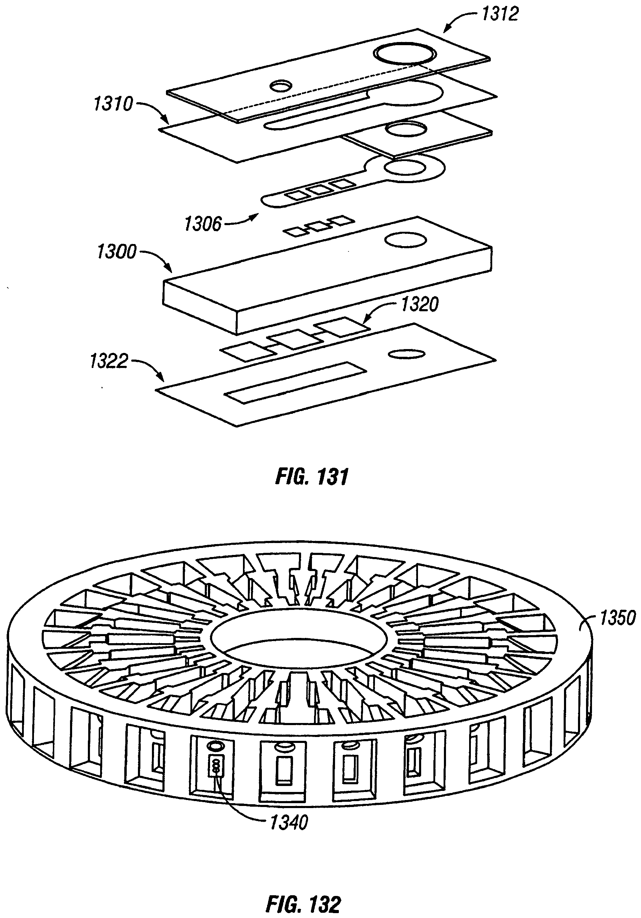

Figure 127 shows the underside of the substrate 1300. Figure 128 shows contact pads 1320 formed on the underside of the substrate 1300. These pads 1320 are in electrical contact with the members 1304. Figure 129 shows a PSA layer 1322 applied over back side with at least one opening 1324 for the contact pads 1320. Figure 130 shows the completed test strip. Figure 131 is a view of the various layers in an exploded perspective view.

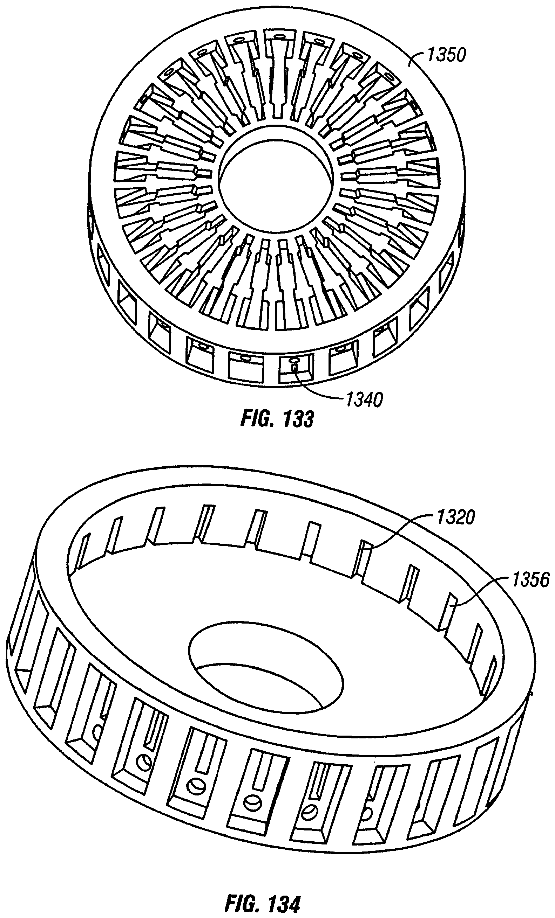

Figures 132 and 133 show how the test strips 1340 may be held in a scaffolding 1350 in a position perpendicular or vertical. The scaffolding 1350 may have openings or slots 1356 to allow for access to the contact pads 1320 on the strip 1340.

While the invention has been described and illustrated with reference to certain particular embodiments thereof, those skilled in the art will appreciate that various adaptations, changes, modifcations, substitutions, deletions, or additions of procedures and protocols may be made.

For example, with any of the above embodiments, the hydrogel may or may not include the mediator.

Expected variations or differences in the results are contemplated in accordance with the objects and practices of the present invention. It is intended, therefore, that the invention be defined by the scope of the claims which follow and that such claims be interpreted as broadly as is reasonable.

## Claims

1. A device comprising:
a plurality of glucose sensing electrodes printed on a substrate having a hydrophobic surface;
a hydrophilic mesh (916) placed on top of the substrate, wherein the mesh has a plurality of fluid channels (920) aligned with the glucose sensing electrodes on the substrate and a plurality of gear teeth extend (918) beyond the edge of the substrate, including one hole per tooth to allow for passage of a lancet;
a sealed and sterilized penetrating member disc (1010) placed on top of the mesh, wherein the disc has a plurality of cavities (306) and a plurality of lancets (302) at least partially contained in said cavities of the sealed and sterilized penetrating member disc wherein the lancets are slidably movable to extend outward from lateral openings on said disc to penetrate tissue, and wherein centerlines of the lancets are directly above the fluid channels and the glucose sending electrodes ; and
a support ring (1020) placed around the circumference of the disc;
wherein the fluid channels are defined by pressure sensitive adhesive impregnated into the mesh;
wherein the substrate and the mesh are bonded together via the pressure sensitive adhesive; wherein the sealed and sterilized penetrating member disc is bonded to the mesh via the pressure sensitive adhesive; and
wherein the gear teeth of the mesh are folded 90 degrees and attached to the edge of the disc and the outer surface of the support ring via the pressure sensitive adhesive and the holes in the teeth are aligned with the lancet centerlines such that the lancets can proceed through the hole unimpeded.

## Patentansprüche

1. Vorrichtung, umfassend:
mehrere Glucose erfassende Elektroden, die auf einem Substrat mit einer hydrophoben Oberfläche gedruckt sind;
einen hydrophilen Zahneingriff (916), der auf das Substrat gelegt ist, wobei der Zahneingriff mehrere Fluidkanäle (920), die auf die Glucose erfassenden Elektroden auf dem Substrat ausgerichtet sind, aufweist und sich mehrere Zahnradzähne über den Rand des Substrats erstrecken (918), einschließlich eines Lochs pro Zahn, um den Durchtritt einer Lanzette zu ermöglichen;
eine abgedichtete und sterilisierte eindringende Gliedscheibe (1010), die auf den Zahneingriff gelegt ist, wobei die Scheibe mehrere Hohlräume (306) und mehrere Lanzetten (302), die mindestens teilweise in den Hohlräumen der abgedichteten und sterilisierten eindringenden Gliedscheibe enthalten sind, aufweist, wobei die Lanzetten gleitend bewegbar sind, um sich von seitlichen Öffnungen auf der Scheibe nach außen zu erstrecken, um in Gewebe einzudringen, und wobei sich Mittellinien der Lanzetten unmittelbar über den Fluidkanälen und den Glucose erfassenden Elektroden befinden; und
einen Trägerring (1020), der um den Umfang der Scheibe gelegt ist;
wobei die Fluidkanäle durch einen druckempfindlichen Kleber, der in den Zahneingriff imprägniert ist, definiert sind;
wobei das Substrat und der Zahneingriff mittels des druckempfindlichen Klebers aneinandergeklebt sind;
wobei die abgedichtete und sterilisierte eindringende Gliedscheibe mittels des druckempfindlichen Klebers an den Zahneingriff geklebt ist; und
wobei die Zahnradzähne des Zahneingriffs um 90 Grad gefaltet und am Rand der Scheibe und an der äußeren Oberfläche des Trägerrings mittels des druckempfindlichen Klebers angebracht sind und die Löcher in den Zähnen derart auf die Mittellinien der Lanzetten ausgerichtet sind, dass die Lanzetten ohne Behinderung durch das Loch voranschreiten können.

## Revendications

1. Dispositif comprenant :
une pluralité d'électrodes de détection de glucose imprimées sur un substrat ayant une surface hydrophobe ;
un treillis hydrophile (916) placé au-dessus du substrat, le treillis ayant une pluralité de canaux de fluide (920) alignés avec les électrodes de détection de glucose sur le substrat et une pluralité de dents de pignon (918) s'étendant au-delà du bord du substrat, comportant un trou par dent pour permettre le passage d'une lancette ;
un disque à éléments pénétrants scellés et stérilisés (1010) placé au-dessus du treillis, le disque ayant une pluralité de cavités (306) et une pluralité de lancettes (302) au moins partiellement contenues dans lesdites cavités du disque à éléments pénétrants scellés et stérilisés, les lancettes étant mobiles par glissement pour s'étendre vers l'extérieur depuis des ouvertures latérales sur ledit disque pour pénétrer un tissu, et les axes des lancettes étant directement au-dessus des canaux de fluide et des électrodes de détection de glucose ; et
une bague de support (1020) placée autour de la circonférence du disque ;
dans lequel les canaux de fluide sont définis par un adhésif sensible à la pression imprégné dans le treillis ;
dans lequel le substrat et le treillis sont collés ensemble par l'adhésif sensible à la pression ;
dans lequel le disque à éléments pénétrants scellés et stérilisés est collé au treillis par l'adhésif sensible à la pression ; et
dans lequel les dents de pignon du treillis sont pliées à 90 degrés et attachées au bord du disque et à la surface extérieure de la bague de support par l'adhésif sensible à la pression et les trous dans les dents sont alignés avec les axes des lancettes de telle sorte que les lancettes peuvent avancer à travers le trou sans entraves.
